# FASCICULE DE BREVET EUROPEEN

(11) **EP 0 989 994 B1**
(45) Date de publication et mention de la délivrance du brevet: **20.04.2005**
(21) Numéro de dépôt: 98940328.2
(22) Date de dépôt: 24.07.1998
(51) Int. Cl.: C07K 5/078, A61K 38/05, A61K 38/08

(54) **DERIVES DE STREPTOGRAMINES, LEUR PREPARATION ET LES COMPOSITIONS QUI LES CONTIENNENT**
STREPTOGRAMINDERIVATE, IHRE HERSTELLUNG UND SIE ENTHALTENDE ZUBEREITUNGEN
STREPTOGRAMINES, THEIR PREPARATION AND COMPOSITIONS CONTAINING THEM

(30) Priorité: 28.07.1997 FR 9709557
(43) Date de publication de la demande: 05.04.2000
(73) Titulaire: Aventis Pharma S.A., 92160 Antony (FR)
(72) Inventeur: COMMERCON, Alain, F-94400 Vitry sur Seine (FR); BOUCHARD, Hervé, F-94320 Thiais (FR); RIBEILL, Yves, Raleigh, NC 27615 (US); BACQUES, Eric, F-91390 Morsang sur Orge (FR); RONAN, Baptiste, F-92140 Clamart (FR); BARRIERE, Jean-Claude, F-91400 Bures sur Yvette (FR); PUCHAULT, Gérard, F-77139 Marcilly (FR); TERRIER, Corinne, F-93190 Livry Gargan (FR)
(86) Numéro de dépôt international: PCT/FR1998/001639
(87) Numéro de publication internationale: WO 1999/005165

(56) Documents cités:
- FR-A- 2 549 065
- GB-A- 2 206 879

## Description

La présente invention concerne des dérivés du groupe A des streptogramines de formule générale : 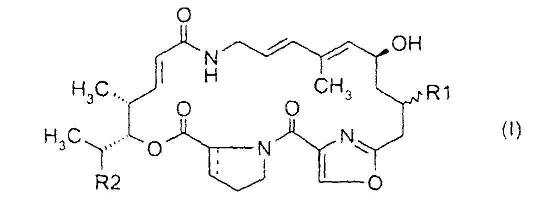 dans laquelle
- R₁ est un radical -NR'R" pour lequel R' est un atome d'hydrogène ou un radical méthyle, et R" est un atome d'hydrogène, un radical alcoyle, cycloalcoyle, allyle, propynyle, benzyle, ou -OR"', R''' étant un atome d'hydrogène, un radical alcoyle, cycloalcoyle, allyle, propynyle ou benzyle, ou R" représente -NR₃R₄, R₃ et R₄ pouvant représenter un radical méthyle, ou former ensemble avec l'atome d'azote auquel ils sont attachés un hétérocycle à 4 ou 5 chaînons saturé ou insaturé pouvant en outre contenir un autre hétéroatome choisi parmi l'azote, l'oxygène ou le soufre,
- R₂ est un atome d'hydrogène ou un radical méthyle ou éthyle, et
- la liaison --- représente une liaison simple ou une liaison double,
ainsi que leurs sels, qui présentent une activité antibactérienne particulièrement intéressante et un bon degré de stabilité métabolique.

Parmi les streptogramines connues, la pristinamycine (RP 7293), antibactérien d'origine naturelle produit par *Streptomyces pristinaespiralis* a été isolée pour la première fois en 1955. La pristinamycine commercialisée sous le nom de Pyostacine® est constituée principalement de pristinamycine IIA associée à la pristinamycine IA.

Un autre antibactérien de la classe des streptogramines : la virginiamycine, a été préparé à partir de *Streptomyces virginiae*, ATCC 13161 [Antibiotics and Chemotherapy, 5, 632 (1955)]. La virginiamycine (Staphylomycine®) est constituée principalement de facteur M₁ associé au facteur S.

Des dérivés hémisynthétiques de streptogramines de structure : 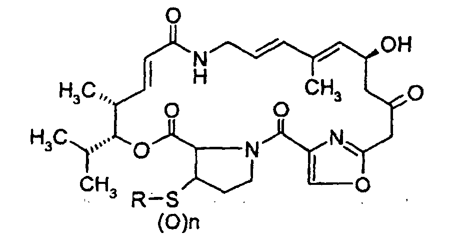 pour lesquels n est 0 à 2 ont été décrits dans les brevets EP 135410 et EP 191662. Associés à une composante hemisynthétique du groupe B des streptogramines, ils manifestent une synergie d'action et sont utilisables par voie injectable.

D'autres dérivés de streptogramines du même type comportant en position 16 un groupement OH, obtenues par réduction de composés correspondants 16 céto, sont décrits dans la demande anglaise GB-A-2 206 879.

Dans la formule générale (I), sauf mention spéciale, les radicaux alcoyle sont droits ou ramifiés et contiennent 1 à 6 atomes de carbone ; les radicaux cycloalcoyle contiennent 3 à 4 atomes de carbone ; la chaîne 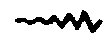 en position 16 signifie : lorsque R" est autre que -OR"' ou -NR₃R₄, l'épimère R ou les mélanges des épimères R et S dans lesquels l'épimère R est majoritaire, et lorsque R" est -OR''' ou -NR₃R₄, les épimères R et S et leurs mélanges.

Lorsque R" est un radical -NR₃R₄ pour lequel R₃ et R₄ forment ensemble avec l'atome d'azote auquel ils sont attachés un hétérocycle à 4 ou 5 chaînons saturé ou insaturé, ce dernier peut être notamment azétidine, azolidine ou imidazolyle.

Les dérivés de streptogramine de formule générale (I) peuvent être préparés à partir des composantes de la pristinamycine naturelle de formule générale : 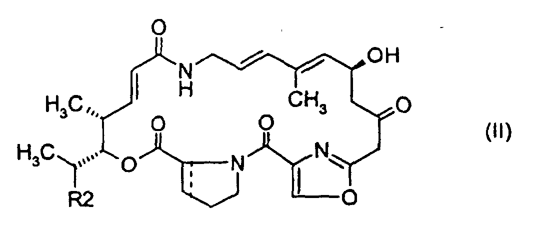 dans laquelle R₂ est défini comme ci-dessus, par action d'une amine de formule générale :

H₂N-R'' (III)

dans laquelle R" est défini comme ci-dessus, suivie de l'action d'un agent réducteur de l'énamine (ou de l'oxime) intermédiaire obtenue, puis, lorsque l'on veut obtenir un dérivé de streptogramine de formule générale (I) pour lequel R' est un radical méthyle, suivie d'une seconde amination réductrice, par action du formaldéhyde ou d'un dérivé générant le formaldéhyde in situ et de la réduction de l'énamine intermédiaire.

L'action de l'amine s'effectue généralement dans un solvant organique comme un alcool (méthanol, éthanol par exemple), un solvant chloré (dichlorométhane, dichloroéthane, chloroforme par exemple), un nitrile (acétonitrile par exemple), la pyridine, à une température comprise entre 0 et 30°C, et éventuellement en présence d'un agent de deshydratation comme par exemple le sulfate de magnésium, le sulfate de sodium ou des tamis moléculaires. De préférence on opère sous atmosphère inerte (argon par exemple). Il est également possible de faire réagir le sel de l'amine.
De préférence, pour préparer des dérivés pour lesquels la liaison --- représente une liaison double, on opère dans un solvant organique comme un nitrile (acétonitrile par exemple) en présence d'un acide, tel qu'un acide organique (acide acétique par exemple) ; dans ce cas l'addition d'un agent de deshydratation n'est pas nécessaire.
Lorsque l'on prépare un dérivé de la streptogramine de formule générale (I) pour lequel R" est un radical -OR''', il est possible d'isoler l'oxime intermédiaire de formule générale: 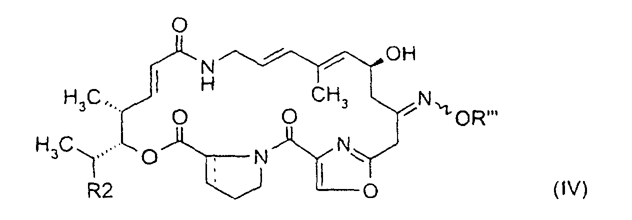 dans laquelle R₂ et R''' sont définis comme ci-dessus, puis de réduire ce produit en un dérivé de formule générale (I) pour lequel R' est un atome d'hydrogène, et éventuellement le mettre en oeuvre dans l'opération subséquente d'amination-réductrice.

La réduction s'effectue par action d'un agent réducteur, par exemple un borohydrure alcalin (cyanoborohydrure ou triacétoxyborohydrure de sodium par exemple) en présence d'un acide organique (acide acétique par exemple) dans un solvant organique tel que cité ci-dessus pour la réaction d'amination.

Le cas échéant, l'opération subséquente d'amination réductrice, destinée à obtenir l'amine disubstituée, s'effectue dans des conditions analogues.

Selon l'invention les dérivés de la streptogramine de formule générale (I) peuvent également être préparés par action de la cétone correspondante au radical R" désiré sur le dérivé aminé de formule générale : 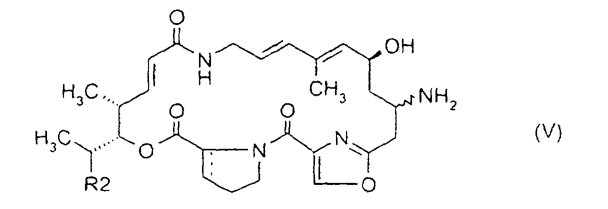 dans laquelle R₂ est défini comme précédemment, suivie lorsque l'on veut obtenir un dérivé de streptogramine de formule générale (I) pour lequel R' est un radical méthyle, d'une seconde amination réductrice, par action du formaldéhyde ou d'un dérivé générant le formaldéhyde in situ et de la réduction de l'énamine intermédiaire.

La réaction s'effectue dans des conditions similaires à celles décrites ci-dessus.

L'amine de formule générale (V) peut être préparée comme décrit précédemment, à partir d'un dérivé de streptogramine de formule générale (II).

Les dérivés de pristinamycine de formule générale (II) correspondent respectivement à la pristinamycine IIA (PIIA), à la pristinamycine IIB (PIIB), à la pristinamycine IIC (PIIC), à la pristinamycine IID (PIID), à la pristinamycine IIF (PIIF) et à la pristinamycine IIG (PIIG) qui sont des composantes connues de la pristinamycine naturelle. Les composantes PIIF et PIIG ont été décrites dans la demande de brevet européen EP 614910.

La pristinamycine IIC (PIIC), et la pristinamycine IID (PIID) peuvent être obtenues comme décrit par J.C. Barrière et coll., Expert. Opin. Invest. Drugs, 3(2), 115-31 (1994).

La préparation et la séparation des composantes des streptogramines naturelles du groupe A [streptogramines de formule générale (II)] est effectuée par fermentation et isolement des constituants à partir du môut de fermentation selon ou par analogie avec la méthode décrite par J. Preud'homme et coll., Bull. Soc, Chim. Fr., vol. 2, 585 (1968).

Alternativement la préparation des composantes naturelles du groupe A peut être effectuée par fermentation spécifique, comme décrit dans la demande de brevet FR 2 689 518.

Les dérivés de streptogramine de formule générale (I) peuvent être purifiés le cas échéant par des méthodes physiques telles que la cristallisation ou la chromatographie.

Les dérivés de formule générale (I) peuvent être notamment obtenus sous la forme épimère 16R. La séparation de la forme épimère 16R et de la forme épimère 16S peut s'effectuer par chromatographie flash, par chromatographie liquide haute performance (CLHP) ou par chromatographie par partage centrifuge (CPC), à partir du mélange des épimères 16R et 16S.

Les dérivés de streptogramine de formule générale (I) peuvent être transformés à l'état de sels d'addition avec les acides, par les méthodes connues. Il est entendu que ces sels entrent aussi dans le cadre de la présente invention.

Comme exemples de sels d'addition avec des acides pharmaceutiquement acceptables, peuvent être cités les sels formés avec les acides minéraux (chlorhydrates, bromhydrates, sulfates, nitrates, phosphates) ou avec les acides organiques (succinates, fumarates, tartrates, acétates, propionates, maléates, citrates, méthanesulfonates, éthanesulfonates, phénylsulfonates, p.toluènesulfonates, iséthionates, naphtylsulfonates ou camphorsulfonates, ou avec des dérivés de substitution de ces composés).

Les dérivés de streptogramine selon la présente invention présentent des propriétés antibactériennes et des propriétés synergisantes de l'activité antibactérienne des dérivés de streptogramine du groupe B. Ils sont particulièrement intéressants du fait de leur activité seuls ou associés ainsi que du fait de leur stabilité métabolique améliorée par comparaison aux dérivés du groupe A antérieurement connus.

Lorsqu'ils sont associés avec une composante ou un dérivé du groupe B des streptogramines, ces derniers peuvent être choisis selon que l'on désire obtenir une forme administrable par voie orale ou parentérale, parmi les composantes naturelles : pristinamycine IA, pristinamycine IB, pristinamycine IC, pristinamycine ID, pristinamycine IE, pristinamycine IF, pristinamycine IG, virginiamycine S1, S3 ou S4, vernamycine B ou C, étamycine ou parmi des dérivés d'hémisynthèse tels que décrits dans les brevets ou demandes de brevet US 4618599, US 4798827, US 5326782, EP 772630 ou EP 770132, notamment des dérivés de streptogramines de formule générale : 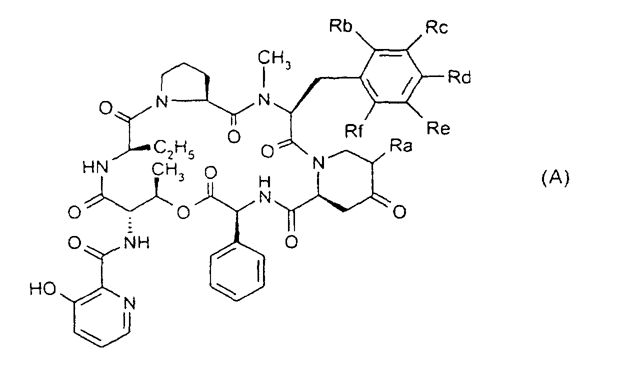 dans laquelle,
1. Rb, Rc, Re et Rf sont des atomes d'hydrogène, Rd est un atome d'hydrogène ou un radical diméthylamino, et Ra est un radical de structure -CH₂R'a pour lequel R'a est pyrrolidinyl-3thio, pipéridyl-3(ou -4)thio pouvant être substitués par alcoyle, alcoylthio substitué par 1 ou 2 hydroxysulfonyle, alcoylamino, dialcoylamino (lui même éventuellement substitué par mercapto ou dialcoylamino), ou substitué par 1 ou 2 cycles pipérazine éventuellement substitué, morpholino, thiomorpholino, pipéridino, pyrrolidinyle-1, pipéridyle-2,-3 ou -4, ou pyrrolidinyle-2 ou -3 (pouvant être substitués par alcoyle), ou bien Ra est un radical de structure =CHR'a pour lequel R'a est pyrrolidinyl-3amino, pipéridyl-3(ou -4)amino, pyrrolidinyl-3oxy, pipéridyl-3(ou -4)oxy, pyrrolidinyl-3thio, pipéridyl-3(ou -4)thio pouvant être substitués par alcoyle, ou R'a est alcoylamino, alcoyloxy ou alcoylthio substitués par 1 ou 2 hydroxysulfonyle, alcoylamino, dialcoylamino (lui même éventuellement substitué par dialcoylamino), ou par trialcoylammonio, imidazolyl-4 ou -5, ou par 1 ou 2 cycles pipérazine éventuellement substitué, morpholino, thiomorpholino, pipéridino, pyrrolidinyle-1, pipéridyle-2,-3 ou -4, ou pyrrolidinyle-2 ou -3 (pouvant être substitués par alcoyle), ou
   Ra est un radical quinuclidinyl-3(ou -4)thiométhyle ou bien
2. Ra est un atome d'hydrogène et
   a) soit Rb, Re et Rf sont des atomes d'hydrogène, Rd est un radical -NHCH₃ ou -N(CH₃)₂ et Rc est un atome de chlore ou de brome, ou représente un radical alcényle contenant 3 à 5 atomes de carbone [si Rd est -N(CH₃)₂],
   b) soit Rb, Rd, Re et Rf représentent un atome d'hydrogène et Rc est un halogène, ou un radical aminomonoalkyle, aminodialkyle, alcoyloxy, trifluorométhyloxy, thioalcoyle,alcoyle en C₁ à C₃ ou trihalogénométhyle
   c) soit Rb, Rc, Re et Rf représentent un atome d'hydrogène et Rd est un halogène, ou un radical éthylamino, diéthylamino ou méthyléthylamino, alcoyloxy ou trifluorométhyloxy, thioalkyle, alcoyle en C₁ à C₆, aryle ou trihalogénométhyle
   d) soit Rb, Re et Rf représentent un atome d'hydrogène et Rc est halogène ou un radical aminomonoalkyle ou aminodialkyle, alcoyloxy ou trifluorométhyloxy, thioalkyle, alcoyle en C₁ à C₃, et Rd est halogène ou un radical amino, aminomonoalkyle ou aminodialkyle, alcoyloxy ou trifluorométhyloxy, thioalkyle, alcoyle en C₁ à C₆ ou trihalogénométhyle.
   e) soit Rc, Re et Rf représentent un atome d'hydrogène et Rb et Rd représentent un radical méthyle.

Il est entendu que les associations des dérivés selon l'invention et des streptogramines du groupe B entrent également dans le cadre de la présente invention.

In vivo, sur les infections expérimentales de la souris à Staphylococcus aureus IP 8203 à des doses comprises entre 25 et 150 mg/kg par voie orale et/ou par voie sous cutanée (DC₅₀), ils synergisent l'activité antimicrobienne de la pristinamycine I_{B}, de la prostinamycine I_{A} ou de la quinupristine (association 30/70).

Enfin, les produits selon l'invention sont particulièrement intéressants du fait de leur faible toxicité. Aucun des produits n'a manifesté de toxicité à des doses de 300 mg/kg ou supérieures à 300 mg/kg par voie sous-cutanée.

D'un intérêt particulier sont les produits de formule générale (I) pour lesquels R₁ est un radical -NR'R" pour lequel R' est un atome d'hydrogène ou un radical méthyle, et R" est un atome d'hydrogène, un radical alcoyle, cycloalcoyle, allyle, propynyle, benzyle, ou -OR"', R''' étant un radical alcoyle contenant 1 à 6 atomes de carbone, allyle ou propynyle, ou R" représente -NR₃R₄, R₃ et R₄ pouvant représenter un radical méthyle, ou former ensemble avec l'atome d'azote auquel ils sont attachés un hétérocycle à 4 ou 5 chaînons saturé ou insaturé pouvant en outre contenir un autre hétéroatome choisi parmi l'azote, l'oxygène ou le soufre, R₂ est un atome d'hydrogène ou un radical méthyle ou éthyle, et la liaison --- représente une liaison simple ou une liaison double, ainsi que leurs sels et dans lesquels la chaîne 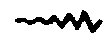 en position 16 signifie : lorsque R" est autre que -OR''' ou -NR₃R₄, l'épimère R ou les mélanges des épimères R et S dans lesquels l'épimère R est majoritaire, et lorsque R" est -OR''' ou -NR₃R₄, les épimères R et S et leurs mélanges ; et parmi ces produits tout particulièrement les dérivés de formule générale (I) pour lesquels R₁ est un radical -NR'R" pour lequel R' est un atome d'hydrogène ou un radical méthyle, et R" est un atome d'hydrogène, un radical alcoyle contenant 1 à 4 atomes de carbone, cycloalcoyle, allyle, propynyle, benzyle, ou -OR''', R''' étant un radical alcoyle contenant 1 à 3 atomes de carbone, allyle ou propynyle, ou R" représente -NR₃R₄, R₃ et R₄ pouvant former ensemble avec l'atome d'azote auquel ils sont attachés un hétérocycle saturé à 5 chaînons, R₂ est un radical méthyle ou éthyle, et la liaison *---* représente une liaison simple ou une liaison double, ainsi que leurs sels et dans lesquels la chaîne 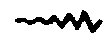 en position 16 est définie comme ci-dessus.

Plus spécialement parmi ces produits, les dérivés suivants sont d'un grand intérêt :
- (16R)-16-Diméthylamino-16-désoxopristinamycine II_{A}.
- (16R)-16-Méthoxyamino-16-désoxopristinamycine II_{B}.
- (16R)-16-Ethoxyamino-16-désoxopristinamycine II_{B:}
- (16R)-16-Allyloxyamino-16-désoxopristinamycine II_{B:}
- (16R)-16-Méthoxyamino-16-désoxopristinamycine II_{A}.

Les exemples suivants, donnés à titre non limitatif, illustrent la présente invention.

Dans les exemples qui suivent, la nomenclature 16-désoxopristinamycine IIA (ou IIB) signifie le remplacement de la fonction cétone en position 16 par 2 atomes d'hydrogène.

### Exemple 1

### (16R)-16-Benzylamino-16-désoxopristinamycine II_{B}

A 1,06 g de pristinamycine II_{B} en solution dans 15 cm³ de méthanol, on ajoute à environ 20°C, sous atmosphère d'argon, 3 g de sulfate de magnésium et 0,328 cm³ de benzylamine. Après 24 heures d'agitation, on ajoute 0,151g de cyanoborohydrure de sodium et 0,5 cm³ d'acide acétique. Le mélange réactionnel est agité 1 heure puis filtré sur Célite. La Célite est lavée par 100 cm³ de méthanol, le filtrat est concentré sous pression réduite (2,7 kPa) pour donner un résidu qui est dilué dans 200 cm³ de dichlorométhane. La phase organique est lavée par 2 fois 100 cm³ d'une solution aqueuse à 5 % en hydrogénocarbonate de sodium. La phase organique est décantée et la phase aqueuse est reprise par 2 fois 50 cm³ de dichlorométhane. Les phases organiques sont réunies, séchées sur sulfate de magnésium, filtrées, puis concentrées à sec sous pression réduite (2,7 kPa), pour donner 1,3 g d'un résidu qui est purifié par chromatographie-flash [éluant : dichlorométhane-méthanol-acétonitrile (84-8-8 en volumes)]. On obtient ainsi 0,424 g d'un mélange des isomères (16R)/(16S)=65/35 de 16-Benzylamino-16-désoxopristinamycine II_{B}, sous forme d'une poudre jaune et 0,144 g du mélange des isomères (16R)/(16S)>95/5 de 16-Benzylamino-16-désoxopristinamycine II_{B} sous forme d'une poudre jaune. Chacun de ces mélanges est purifié par CLHP [colonne C18 (15-20 µm), (λ=254 nm), éluant : acétonitrile-eau (80-20 en volumes)], pour donner, au total, 0,250 g de (16R)-16-benzylamino-16-désoxopristinamycine II_{B} sous forme d'une poudre jaune pâle fondant vers 130°C (déc.).

Spectre de R.M.N. ¹ H (400 MHz, CDCl₃, δ en ppm) : 0,95 et 1,00 (2 d, J = 6,5 Hz, 3H chacun : CH₃ en 30 et CH₃ en 31) : 1,08 (d, J = 6,5 Hz, 3H : CH₃ en 32) : 1,43 (mt, 1H : 1H du CH₂ en 15) ; 1,68 (s, 3H : CH₃ en 33) ; de 1,70 à 2,00 (mt, 5H : 1H du CH₂ en 15 - CH₂ en 25 - 1H du CH₂ en 26 et CH en 29) ; 2,12 (mt, 1H : 1H du CH₂ en 26) ; de 2,65 à 2,80 (mt, 1H : CH en 4) ; 2,78 et 3,18 (2 dd, respectivement J = 16 et 8 Hz et J = 16 et 4 Hz. 1H chacun : CH₂ en 17) ; 3,25 (mt, 1H : CH en 16) ; 3.47(mt, 1H : 1H du CH₂ en 9) ; 3,80 (mt, 1H : 1H du CH₂ en 24) ; 3.81 et 4,02 (2 d, J = 14 Hz, 1H chacun : CH₂N) ; 3,96 (mt, 1H : 1H du CH₂ en 24) ; 4,38 (mt, 1H : 1H du CH₂ en 9) ; 4,66 (mt, 1H : CH en 14) ; de 4,70 à 4,80 (mt, 2H : CH en 3 et CH en 27) ; 5,36 (d, J = 9 Hz, 1H : CH en 13) ; 5,64 (mt, 1H : CH en 10) ; 5,78 (dd, J = 16 et 2 Hz, 1H : CH en 6) ; 6,00 (mt , 1H : CONH) ; 6,14 (d, J = 16 Hz, 1H : CH en 11) ; 6,50 (dd, J = 16 et 5 Hz, 1H : CH en 5) ; de 7,25 à 7,40 (mt, 5H : H aromatiques du benzyle) ; 8,09 (s, 1H : CH en 20).

### Exemple 2

### (16R)-16-Cyclopropylamino-16-désoxopristinamycine II_{B}

A 3 g de pristinamycine II_{B} en solution dans 30 cm³ de méthanol, on ajoute à environ 20°C, sous atmosphère d'argon, 9 g de sulfate de magnésium et 0,6 cm³ de cyclopropylamine. Après 17 heures 30 minutes d'agitation, on ajoute 0,43 g de cyanoborohydrure de sodium puis après 30 minutes 0,5 cm³ d'acide acétique. Le mélange réactionnel est agité 2 heures puis filtré sur Célite. La Célite est lavée au méthane) puis le filtrat est concentré sous pression réduite (2,7 kPa) pour donner 4,64 g d'une meringue jaune qui est dissoute dans 100 cm³ d'acétate d'éthyle et 5 cm³ de méthanol puis lavée par 3 fois 25 cm³ d'eau distillée. Les phases organiques sont réunies, séchées sur sulfate de magnésium, filtrées puis concentrées sous pression réduite (2,7 kPa) pour donner 2,2 g d'une meringue jaune qui est purifiée par chromatographie-flash [éluant : dichlorométhane-méthanol-acétonitrile (84-8-8 en volumes)]. On isole 0,61 g d'une poudre jaune qui est agitée dans 15 cm³ d'éther éthylique, filtrée puis séchée sous pression réduite (2,7 kPa), à 30°C, pour donner 0,508 g de (16R)-16-cyclopropylamino-16-désoxopristinamycine II_{B} sous forme d'une poudre crème fondant vers 135°C (déc.).

Spectre de R.M.N. ¹ H (400 MHz, CDCl₃, δ en ppm) : 0,35 à 0,60 (mt, 4H : CH₂ du cyclopropyle) ; 0,97 et 1,00 (2 d, J = 6,5 Hz, 3H chacun : CH₃ en 30 et CH₃ en 31) ; 1,08 (d, J = 6,5 Hz, 3H : CH₃ en 32) ; 1,33 (mt, 1H : 1H du CH₂ en 15) ; de 1,70 à 2,00 (mt, 5H : 1H du CH₂ en 15 - CH₂ en 25 - 1H du CH₂ en 26 et CH en 29) ; 1,77 (s, 3H : CH₃ en 33) ; 2,13 (mt, 1H : 1H du CH₂ en 26) ; 2,29 (mt, 1H : CH du cyclopropyle) ; 2,74 (mt, 1H : CH en 4) ; 2,82 et 3,25 (2 dd, respectivement J = 16 et 8 Hz et J = 16 et 4 Hz, 1H chacun : CH₂ en 17) ; 3,33 (mt, 1H : CH en 16) ; 3,51 (mt, 1H : 1H du CH₂ en 9) ; 3,83 et 3,99 (2 mts, 1H chacun : CH₂ en 24) ; 4,35 (mt, 1H : 1H du CH₂ en 9) ; 4,65 (mt, 1H : CH en 14) ; de 4,70 à 4,80 (mt, 2H : CH en 3 et CH en 27) ; 5,39 (d, J = 9 Hz, 1H : CH en 13) ; 5,65 (mt, 1H : CH en 10) ; 5,79 (dd, J = 17 et 2 Hz, 1H : CH en 6) ; 5,97 (mt, 1H : CONH) ; 6,17 (d, J = 16 Hz, 1H : CH en 11) ; 6,53 (dd, J = 17 et 5 Hz, 1H : CH en 5) ; 8,12 (s, 1H : CH en 20).

### Exemple 3

### (16R)-16-Allylamino-16-désoxopristinamycine II_{B}

En opérant d'une manière analogue à celle décrite à l'exemple 1, mais à partir de 5 g de pristinamycine II _{B} en solution dans 70 cm³ de méthanol, 15 g de sulfate de magnésium et de 1,45 cm³ d'allylamine et après ajout à 24 heures de 0,714 g de cyanoborohydrure de sodium et de 5 cm³ d'acide acétique, on obtient après 1 heure d'agitation supplémentaire et après traitement, un solide qui est purifié par chromatographie- flash [éluant : dichlorométhane-méthanol (95-5 en volumes)], pour donner 0,975 g de (16R)-16-allylamino-16-désoxopristinamycine II_{B}, sous forme d'une poudre jaune pâle fondant vers 122-124°C.

Spectre de R.M.N. ¹ H (400 MHz, CDCl₃, δ en ppm) : 0,96 et 1,00 (2 d, J = 6,5 Hz, 3H chacun : CH₃ en 30 et CH₃ en 31) ; 1,08 (d, J = 6,5 Hz, 3H : CH₃ en 32) ; 1,38 (mt, 1H : 1H du CH₂ en 15) ; de 1,65 à 2,00 (mt, 5H : 1H du CH₂ en 15 - CH₂ en 25 - 1H du CH₂ en 26 et CH en 29) ; 1.77 (s, 3H : CH₃ en 33) ; 2,12 (mt, 1H : 1H du CH₂ en 26) ; de 2,70 à 2,80 (mt, 1H : CH en 4) ; 2,74 et 3,13 (2 dd, respectivement J = 16 et 8 Hz et J = 16 et 5 Hz, 1H chacun : CH₂ en17) ; de 3,20 à 3,35 (mt, 2H : CH en 16 et 1H du CH₂N) ; de 3,45 à 3,55 (mt, 2H : 1H du CH₂ en 9 et 1H du CH₂N) ; 3,83 et 3,98 (2 mts, 1H chacun : CH₂ en 24) ; 4,38 (mt, 1H : 1H du CH₂ en 9) ; 4,70 (mt, 1H : CH en 14) ; de 4,65 à 4,80 (mt, 2H : CH en 3 et CH en 27) ; 5,15 et 5,24 (2 dd, respectivement J = 10 et 1,5 Hz et J = 18 et 1,5 Hz, 1H chacun : =CH₂ de l'allyle) ; 5,40 (d, J = 9 Hz, 1H : CH en 13) ; 5,66 (mt, 1H : CH en 10) ; 5,80 (dd, J = 17 et 2,5 Hz, 1H : CH en 6) ; de 5,85 à 6,00 (mt, 2H : CH= de l'allyle et CONH) : 6,16 (d, J = 16 Hz, 1H : CH en 11) ; 6,52 (dd, J = 17 et 5 Hz, 1H : CH en 5) ; 8,10 (s, 1H : CH en 20).

### Exemple 4

### (16R)-16-Propyne-2-ylamino-16-désoxopristinamycine II_{B}

En opérant d'une manière analogue à celle décrite à l'exemple 1, mais à partir de 5 g de pristinamycine II _{B} en solution dans 70 cm³ de méthanol, de 10 g de sulfate de magnésium et de 1,3 cm³ de propargylamine et après ajout à 22 heures de 0,714 g de cyanoborohydrure de sodium et de 5 cm³ d'acide acétique, on obtient après 3 heures 30 mn d'agitation supplémentaire et après traitement, 5,5 g d'un solide qui est purifié par chromatographie-flash [éluant : dichlorométhane-méthanol (96-4 en volumes)], pour 0,266 g de (16R)-16-Propyne-2-ylamino-16-désoxopristinamycine II_{B}, sous forme d'une poudre ocre fondant vers 124°C (déc.).

Spectre de R.M.N. ¹ H (400 MHz, CDCl₃, δ en ppm) : 0,95 et 1,00 (2 d, J = 6,5 Hz, 3H chacun : CH₃ en 30 et CH₃ en 31) ; 1,07 (d, J = 6.5 Hz, 3H : CH₃ en 32) ; 1,53 (mt, 1H : 1H du CH₂ en 15) ; de 1,60 à 2,00 (mt, 5H : 1H du CH₂ en 15 - CH₂ en 25 - 1H du CH₂ en 26 et CH en 29) ; 1,79 (s, 3H : CH₃ en 33) ; 2,13 (mt, 1H : 1H du CH₂ en 26) ; 2,26 (t, J = 2 Hz, 1H : CH propynyle) ; de 2,70 à 2,80 (mt, 1H : CH en 4) ; 2,76 et 3,16 (2 dd, respectivement J = 16 et 8 Hz et J = 16 et 4 Hz, 1H chacun : CH₂ en 17) ; 3,36 (mt, 1H : CH en 16) ; 3,48 (mt, 1H : 1H du CH₂ en 9) ; 3,56 (AB limite, 2H : NCH₂ propynyle) ; 3,84 et 3,99 (2 mts, 1H chacun : CH₂ en 24) ; 4,40 (mt, 1H : 1H du CH₂ en 9) ; de 4,65 à 4,80 (mt, 3H : CH en 3 - CH en 14 et CH en 27) ; 5,36 (d, J = 9 Hz, 1H : CH en 13) ; 5,69 (mt, 1H : CH en 10) ; 5,80 (dd, J = 16 et 2 Hz, 1H: CH en 6) ; 6,11 (mt, 1H : CONH) ; 6,17 (d, J = 16 Hz, 1H : CH en 11) ; 6,52 (dd, J = 16 et 5 Hz, 1H : CH en 5) ; 8.08 (s, 1H : CH en 20).

### Exemple 5

### (16R)-16-[(R)-sec-Butylamino]-16-désoxopristinamycine II_{B}

En opérant d'une manière analogue à celle décrite à l'exemple 1, mais à partir de 5 g de pristinamycine II _{B} en solution dans 70 cm³ de méthanol, de 10 g de sulfate de magnésium et de 1,92 cm³ de (R)-sec-butylamine et après ajout à 20 heures d'agitation de 0,714 g de cyanoborohydrure de sodium et de 5 cm³ d'acide acétique, on obtient après 2 heures 30 mn d'agitation supplémentaire et après traitement 5,6 g d'un solide qui est purifié par chromatographie-flash [éluant : dichlorométhane-méthanol (96-4 en volumes)], pour donner 0,680 g de (16R)-16-[(R)-sec-butylamino]-16-désoxopristinamycine II_{B}, sous forme d'une poudre jaune fondant vers 156°C (déc.).

Spectre de R.M.N. ¹ H (400 MHz, CDCl₃, δ en ppm) : de 0,90 à 1,15 (mt, 15H : CH₃ en 30 - CH₃ en 31 - CH₃ en 32 et 2 CH₃ de (N-2-Butyle)) ; 1,28 (mt, 2H : CH₂ de (N-1-Méthylpropyle)) ; 1,50 à 2,20 (mt, 7H : CH₂ en 15 - CH₂ en 25 - CH₂ en 26 et CH en 29) ; 1,79 (s, 3H : CH₃ en 33) ; 2,74 (mt, 1H : CH en 4) ; de 3,00 à 3,10 (mt, 2H : CH de (N-1-Méthylpropyle) et 1H du CH₂ en 17) ; 3,25 (dd, J = 16 et 4 Hz, 1H : 1H du CH₂ en 17) ; de 3,50 à 3,60 (mt, 2H : 1H du CH₂ en 9 et CH en 16) ; 3,80 et 3,95 (2 mts, 1H chacun : CH₂ en 24) ; 4,28 (mt, 1H : 1H du CH₂ en 9) ; de 4,70 à 4,85 (mt, 3H : CH en 3 - CH en 14 et CH en 27) ; 5,41 (d, J = 9 Hz, 1H : CH en 13) ; 5,68 (mt, 1H : CH en 10) ; 5,80 (d, J = 16 Hz, 1H : CH en 6) ; de 6,10 à 6,25 (mf étalé, 1H : CONH) ; 6,18(d,J = 16 Hz, 1H : CH en 11) ; 6,55 (dd, J = 17 et 5 Hz, 1H : CH en 5); 8,11 (s, 1H : CH en 20).

### Exemple 6

### (16R)-16-[(S)-sec-Butylamino]-16-désoxopristinamycine II_{B}

En opérant d'une manière analogue à celle décrite à l'exemple 1, mais à partir de 5 g de pristinamycine II _{B} en solution dans 70 cm³ de méthanol, de 10 g de sulfate de magnésium et de 1,92 cm³ de (S)-sec-butylamine et en ajoutant après 20 heures d'agitation 0,714 g de cyanoborohydrure de sodium et 5 cm³ d'acide acétique. Le mélange réactionnel est agité 2 heures 30 mn et conduit, après traitement, à un solide qui est purifié par chromatographie-flash [éluant : dichlorométhane-méthanol (96-4 en volumes)]. Le solide obtenu est recristallisé dans l'acétonitrile chaud, pour donner 0,590 g de (16R)-16-[(S)-sec-butylamino]-16-désoxopristinamycine II_{B} sous forme d'une poudre jaune fondant vers 150°C (déc.).

Spectre de R.M.N. ¹ H (400 MHz, CDCl₃, δ en ppm) : de 0,90 à 1,15 (mt, 15H : CH₃ en 30 - CH₃ en 31 - CH₃ en 32 et 2 CH₃ de (N-1-Méthylpropyle)) ; 1,44 (quintuplet, J = 7 Hz, 2H : CH₂ de (N-1-Méthylpropyle)) ; 1,60 à 2,00 (mt, 5H : 1H du CH₂ en 15 - CH₂ en 25 - 1H du CH₂ en 26 et CH en 29) ; 1,76 (s, 3H : CH₃ en 33) ; 2,04 (d large, J = 14 Hz, 1H : 1H du CH₂ en 15) ; 2,11 (mt, 1H : 1H du CH₂ en 26) ; de 2,65 à 2,85 (mt, 1H : CH en 4) ; 2,70 et 3,12 (2 dd, respectivement J = 16 et 11 Hz et J = 16 et 4 Hz. 1H chacun : CH₂ en 17) ; 2,80 (mt, 1H : CH de (N-1-Méthylpropyle)) : 3,35 (mt, 1H : CH en 16) ; 3,50 (mt, 1H : 1H du CH₂ en 9) ; 3,81 et 3,98 (2 mts, 1H chacun : CH₂ en 24) ; 4,33 (mt, 1H : 1H du CH₂ en 9) ; de 4,65 à 4,80 (mt, 3H : CH en 3 - CH en 14 et CH en 27) ; 5,43 (d, J = 9 Hz, 1H : CH en 13) ; 5,62 (mt, 1H : CH en 10) ; 5,78 (dd, J = 16 et 2 Hz, 1H : CH en 6) ; 5,86 (mt, 1H : CONH) ; 6,17 (d, J = 16 Hz, 1H : CH en 11) ; 6,53 (dd, J = 17 et 5 Hz, 1H : CH en 5) ; 8,12 (s, 1H : CH en 20).

### Exemple 7

### 16-Amino-16-désoxopristinamycine II_{B}

### [mélange des isomères (16R)/(16S)=75/25] :

A 100 g de pristinamycine II_{B} en solution dans 1400 cm³ de méthanol, on ajoute à environ 20°C sous atmosphère d'argon, 200 g de sulfate de magnésium, 74 g d'acétate d'ammonium et 28 g de cyanoborohydrure de sodium. Après 20 heures d'agitation, le mélange réactionnel est filtré sur Célite, puis la Célite rincée au méthanol. Le filtrat est concentré sous pression réduite (2,7 kPa) pour donner une huile marron qui est partagée en deux fractions égales qui sont chacune diluées dans 1000 cm³ de dichlorométhane puis traitées par une solution aqueuse à 5 % de bicarbonate de sodium. Les phases organiques sont décantées et les phases aqueuses extraites par 1000 cm³ de dichlorométhane. Les phases organiques sont réunies, lavées par une solution aqueuse saturée en chlorure de sodium, séchées sur sulfate de magnésium, filtrées puis concentrées à sec sous pression réduite (2,7 kPa), pour donner 97,5 g d'une poudre jaune foncé. Celle-ci est purifiée par chromatographie-flash [éluant : dichlorométhane-méthanol (70-30 en volumes)], pour donner 21,7 g de 16-amino-16-désoxopristinamycine II_{B} (mélange des isomères (16R)/(16S)=75/25), sous forme d'une poudre jaune beige.

Spectre de R.M.N. ¹ H [ isomère Syn (16R) 75% isomère Anti (16S) 25%] (400 MHz, CDCl₃, δ en ppm) : 0,94 et 0,98 (2d, J = 6,5 Hz : CH₃ en 30 et CH₃ en 31 de l'isomère Syn) ; de 0.90 à 1,15 (mt : CH₃ en 30 - CH₃ en 31 et CH₃ en 32 de l'isomère Anti ) ; 1,07 (d, J = 6,5 Hz : CH₃ en 32 de l'isomère Syn) ; 1,46 (mt : 1H du CH₂ en 15 de l'isomère Syn) ; de 1,65 à 2,00 (mt : 1H du CH₂ en 15 de l'isomère Syn - 1H du CH₂ en 15 de l'isomère Anti - CH₂ en 25 - 1H du CH₂ en 26 et CH en 29) ; 1,73 et 1,78 (2 s : respectivement CH₃ en 33 de l'isomère Anti et CH₃ en 33 de l'isomère Syn) ; de 2,00 à 2,30 (mt : 1H du CH₂ en 15 de l'isomère Anti et 1H du CH₂ en 26) ; de 2,65 à 2,80 (mt : CH en 4 et 1H du CH₂ en 17 de l'isomère Syn) ; de 2,80 à 2,90 (mt : CH₂ en 17 de l'isomère Anti ) ; 2,95 (dd, J = 16 et 5 Hz : 1H du CH₂ en 17 de l'isomère Syn) ; de 3,30 à 3,45 (mt : 1H du CH₂ en 9 de l'isomère Anti et CH en 16 de l'isomère Syn) ; 3,48 (mt : 1H du CH₂ en 9 de l'isomère Syn) ; 3,63 (mt: CH en 16 de l'isomère Anti ) : 3,80 et 3,91 (2 mts : respectivement CH₂ en 24 de l'isomère Anti et CH₂ en 24 de l'isomère Syn) ; 4,37 (mt : 1H du CH₂ en 9 de l'isomère Syn) ; 4,45 (mt : 1H du CH₂ en 9 de l'isomère Anti ) ; de 4,65 à 4,80 (mt : CH en 3 - CH en 27 et CH en 14 de l'isomère Syn) ; 4,85 (mt : CH en 14 de l'isomère Anti ) ; 5,40 (d, J = 9 Hz : CH en 13 de l'isomère Syn) ; 5,65 (mt : CH en 10) ; de 5,70 à 5,85 (mt : CH en 6 et CH en 13 de l'isomère Anti ) ; de 6,00 à 6,15 (mt : CONH) ; 6,18 et 6,22 (2 d, J =16 Hz, respectivement CH en 11 de l'isomère Syn et CH en 11 de l'isomère Anti ) ; 6,45 et 6,52 (2 dd, J = 16 et 5 Hz : respectivement CH en 5 de l'isomère Anti et CH en 5 de l'isomère Syn) ; 8,09 et 8,10 (s : respectivement CH en 20 de l'isomère Syn et CH en 20 de l'isomère Anti ).

Par chromatographie liquide haute performance à partir de 16-Amino-16-désoxopristinamycine II_{B} (mélange des isomères (16R)/(16S)=75/25), on obtient la (16R)-16-amino-16-désoxopristinamycine II_{B} sous forme d'une poudre blanche fondant vers 130°C (déc.).

Spectre de R.M.N. ¹ H (400 MHz, CDCl₃, δ en ppm) : 0,95 et 0,99 (2 d, J = 6,5 Hz, 3H chacun : CH₃ en 30 et CH₃ en 31) ; 1,08 (d, J = 6,5 Hz, 3H : CH₃ en 32) ; 1,45 (mt, 1H : 1H du CH₂ en 15) ; de 1,65 à 2,00 (mt, 5H : 1H du CH₂ en 15 - CH₂ en 25 - 1H du CH₂ en 26 et CH en 29) ; 1,79 (s, 3H : CH₃ en 33) ; 2,12 (mt, 1H : 1H du CH₂ en 26) ; de 2,70 à 2,80 (mt, 1H : CH en 4) ; 2,76 et 2,98 (2 dd, respectivement J = 16 et 8 Hz et J = 16 et 5 Hz, 1H chacun : CH₂ en 17) ; 3,42 (mt, 1H : CH en 16) ; 3,48 (mt, 1H : 1H du CH₂ en 9) ; 3,93 (mt, 2H : CH₂ en 24) ; 4,40 (mt, 1H : 1H du CH₂ en 9) ; de 4,70 à 4,80 (mt, 3H : CH en 3 - CH en 14 et CH en 27) ; 5,42 (d, J = 9 Hz, 1H : CH en 13) ; 5,67 (mt, 1H : CH en 10) ; 5,79 (dd, J = 17 et 2,5 Hz, 1H : CH en 6) ; 5,93 (mt, 1H : CONH) ; 6,18 (d, J = 16 Hz, 1H : CH en 11) ; 6,52 (dd, J = 17 et 5 Hz, 1H : CH en 5) ; 8,12 (s, 1H : CH en 20).

### Exemple 8

### 16-Méthylamino-16-désoxopristinamycine II_{B}

### [mélange des isomères (16R)/ (16S) = 70/30] :

A 20 g de pristinamycine II_{B} en solution dans 280 cm³ de méthanol, on ajoute à environ 20°C, sous atmosphère d'argon, 56 g de sulfate de magnésium et 9,46 cm³ de méthylamine en solution dans l'éthanol (environ 8 M). Le mélange réactionnel est agité à environ 20°C durant 24 heures. Le mélange réactionnel est alors filtré sur Célite, puis la Célite lavée plusieurs fois par du méthanol. On ajoute alors au filtrat 2,86 g de cyanoborohydrure de sodium et 9,46 cm³ d'acide acétique. Après agitation du mélange réactionnel durant 5 heures, la solution obtenue est concentrée à sec, sous pression réduite (2,7 kPa), à 30°C. Le résidu est dissout dans 200 cm³ de dichlorométhane puis lavée par une solution aqueuse saturée de bicarbonate de sodium. Les phases aqueuses sont décantées puis extraites par 3x100 cm³ de dichlorométhane. Les phases organiques sont réunies, séchées sur sulfate de magnésium, filtrées puis concentrées sous pression réduite (2,7 kPa) pour donner 17,8 g d'une poudre orange qui est purifiée par chromatographie-flash [éluant : dichlorométhane-méthanol (70-30 puis 60/40 en volumes)]. On isole ainsi 10,5 g de 16-méthylamino-16-désoxopristinamycine II_{B} (mélange des isomères (16R)/(16S) = 70/30), sous forme d'une poudre jaune.

Spectre de R.M.N. ¹ H [isomère Syn (16R) 70% , isomère Anti (16S) 30%] (400 MHz, CDCl₃, δ en ppm) : de 0,90 à 1,10 (mt, 9H : CH₃ en 30 - CH₃ en 31 et CH₃ en 32) ; de 1,20 à 1,40 (mt : 1H du CH₂ en 15 de l'isomère Syn) ; de 1,65 à 2,00 (mt : 1H du CH₂ en 15 de l'isomère Syn - 1H du CH₂ en 15 de l'isomère Anti - CH₂ en 25 - 1N du CH₂ en 26 et CH en 29) ; 1,73 et 1,77 (2 s : respectivement CH₃ en 33 de l'isomère Anti et CH₃ en 33 de l'isomère Syn) ; de 2,05 à 2,15 (mt : 1H du CH₂ en 26) ; 2,18 (dt, J = 15 et 3 Hz, 1H du CH₂ en 1 de l'isomère Anti ) ; de 2,20 à 2,60 (mf étalé : NH) ; 2,51 et 2,52 (2 s : respectivement NCH₃ de l'isomère Anti et NCH₃ de l'isomère Syn) ; 2.60 (dd, J = 16 et 11 Hz : 1H du CH₂ en 17 de l'isomère Anti ) : de 2,70 à 2,80 (mt : CH en 4) ; 2,75 (dd, J = 16 et 8 Hz : 1H du CH₂ en 17 de l'isomère Syn) ; de 3,05 à 3,20 (mt : 1H du CH₂ en 17 et CH en 16 de l'isomère Syn) ; de 3,30 à 3,40 (mt : 1H du CH₂ en 9 de l'isomère Anti et CH en 16 de l'isomère Anti ) ; 3,48 (mt : 1H du CH₂ en 9 de l'isomère Syn) ; 3,83 et 3,98 (2 mts, 2 heures en totalité : CH₂ en 24) ; 4.37 (mt : 1H du CH₂ en 9 de l'isomère Syn) ; 4,50 (mt : 1H du CH₂ en 9 de l'isomère Anti ) ; de 4,65 à 4,80 (mt : CH en 3 - CH en 27 et CH en 14 de l'isomère Syn) ; 4,83 (mt : CH en 14 de l'isomère Anti ) ; 5,39 (d, J = 9 Hz : CH en 13 de l'isomère Syn) : 5,65 (mt, 1H : CH en 10) ; de 5,70 à 5,85 (mt : CH en 6 et CH en 13 de l'isomère Anti ) ; 5,96 (mt, 1H : CONH) ; 6,16 et 6,23 (2 d, J = 16 Hz, 1H en totalité : respectivement CH en 11 de l'isomère Syn et CH en 11 de l'isomère Anti ) ; 6,45 et 6,52 (2 dd, J = 16 et 5 Hz, 1H en totalité : respectivement CH en 5 de l'isomère Anti et CH en 5 de l'isomère Syn) ; 8,10 et 8,12 (s : respectivement CH en 20 de l'isomère Syn et CH en 20 de l'isomère Anti ).

Par chromatographie liquide haute performance à partir de 16-Méthylamino-16-désoxopristinamycine II_{B} (mélange des isomères (16R)/(16S) = 70/30), on obtient (16R)-16-méthylamino-16-désoxopristinamycine II_{B} sous forme d'un solide jaune fondant vers 128°C (déc.).

Spectre de R.M.N. ¹ H (400 MHz, CDCl₃, δ en ppm) : 0,96 et 1,00 (2 d, J = 6,5 Hz, 3H chacun : CH₃ en 30 et CH₃ en 31) ; 1,08 (d, J = 6,5 Hz, 3H : CH₃ en 32) ; 1,33 (mt, 1H : 1H du CH₂ en 15) ; de 1,60 à 2,05 (mt, 5H : 1H du CH₂ en 15 - CH₂ en 25 - 1H du CH₂ en 26 et CH en 29) ; 1,75 (s, 3H : CH₃ en 33) ; 2,11 (mt, 1H : 1H du CH₂ en 26) ; 2,53 (s, 3H : NCH₃) ; 2,70 à 2,80 (mt, 1H : CH en 4) ; 2,75 et 3,12 (2 dd, respectivement J = 16 et 8 Hz et J = 16 et 4 Hz, 1H chacun : CH₂ en17) ; 3,10 à 3,20 (mt, 1H : CH en 16) ; 3,48 (mt, 1H : 1H du CH₂ en 9) ; 3,82 et 3,98 (2 mts, 1H chacun : CH₂ en 24) ; 4,37 (mt, 1H : 1H du CH₂ en 9) ; de 4,65 à 4,80 (mt, 3H : CH en 3 - CH en 14 et CH en 27) ; 5,40 (d, J = 9 Hz, 1H : CH en 13) ; 5,64 (mt, 1H : CH en 10) ; 5,78 (dd, J = 17 et 2,5 Hz, 1H : CH en 6) ; 5,96 (mt, 1H : CONH) ; 6,16 (d, J= 16 Hz, 1H : CH en 11) ; 6,52 (dd, J = 17 et 5 Hz, 1H : CH en 5) ; 8,10 (s, 1H : CH en 20).

### Exemple 9

### Chlorhydrate de (16R)-16-Isopropylamino-16-désoxopristinamycine II_{B}

A 12 g de pristinamycine II_{B} en solution dans 120 cm³ de méthanol, on ajoute à environ 20°C sous atmosphère d'argon, 72 g de sulfate de magnésium, 8,8 g d'acétate d'ammonium et 3,3 g de cyanoborohydrure de sodium. Après 20 heures d'agitation, on ajoute 33,5 cm³ d'acétone et le mélange réactionnel est agité 7 heures à environ 20°C avant filtration sur Célite. La Célite est lavée plusieurs fois par du dichlorométhane et les filtrats rassemblés sont concentrés à sec, sous pression réduite (2,7 kPa), à 30°C. Le résidu est dissout dans 400 cm³ de dichlorométhane et la solution ainsi obtenue est lavée par 3x200 cm³ d'une solution aqueuse saturée de bicarbonate de sodium. La phase organique finale est concentrée à sec, sous pression réduite (2,7 kPa), à 30°C, pour donner 11,3 g d'une poudre orange qui est purifiée par chromatographie-flash [éluant en gradient : n-butanol-acétate d'éthyle-éthanol-eau (10-60-15-15 puis 20-50-15-15 puis 30-40-15-15 en volumes)] pour donner 2,92 g de 16-Isopropylamino-16-désoxopristinamycine II_{B} (mélange des isomères (16R)/(16S)=75/25), sous forme d'une poudre jaune. Les deux épimères sont séparés par chromatographie de partage centrifuge [éluant : acétate d'éthyle-hexane-méthanol-eau (2-1-1-1,8 en volumes)], pour donner 0,77 g de (16R)-16-Isopropylamino-16-désoxopristinamycine II_{B}, sous forme d'une poudre blanche. Celle-ci est dissoute dans un mélange de 10,8 cm³ d'acide chlorhydrique 0,1 N et de 27,7 cm³ d'eau. La solution ainsi obtenue est alors filtrée et le filtrat lyophilisé pour donner 0,72 g de chlorhydrate de (16R)-(6-isopropylamino-16-désoxopristinamycine II_{B}, sous forme d'une poudre blanche fondant vers 135°C (déc.).

Spectre de R.M.N. ¹ H (400 MHz, CDCl₃, δ en ppm) : de 0,90 à 1,05 (mt, 6H : CH₃ en 30 et CH₃ en 31) ; 1,07 (d, J = 6,5 Hz, 3H : CH₃ en 32) ; 1,44 et 1,54 (2 d, J = 6,5 Hz, 3H chacun : 2 CH₃ de l'isopropyle) ; de 1,70 à 2,25 (mt, 7H : CH₂ en 15 - CH₂ en 25 - CH₂ en 26 et CH en 29) ; 1,85 (s, 3H : CH₃ en 33) ; 2,74 (mt, 1H : CH en 4) ; 3,34 (mt, 2H : CH₂ en 17) ; 3,45 (mt, 1H : CH de l'isopropyle) ; 3,56 (mt, 1H : 1H du CH₂ en 9) ; 3,67 (mt, 1H : CH en 16) ; 3,82 et 3,95 (2 mts, 1H chacun : CH₂ en 24) ; 4,31 (mt, 1H : 1H du CH₂ en 9) ; de 4,70 à 4,80 (mt, 2H : CH en 3 et CH en 27) : 4,85 (mt, 1H : CH en 14) ; 5,41 (d, J = 9 Hz, 1H : CH en 13) ; 5,75 (mt, 1H : CH en 10) ; 5,83 (dd, J = 16 et 2 Hz, 1H : CH en 6) ; 6,22 (d, J = 16 Hz, 1H : CH en 11) ; 6,46 (mt, 1H : CONH) ; 6,54 (dd, J = 16 et 5 Hz, 1H : CH en 5) ; 8,10 (s : CH en 20).

### Exemple 10

### (16R)-16-Diméthylamino-16-désoxopristinamycine II_{B}

A 20 g de pristinamycine II_{B} en solution dans 300 cm³ de méthanol, on ajoute à environ 20°C, sous atmosphère d'argon, 50 de sulfate de magnésium et 9,5 cm³ de méthylamine. Après 22 heures d'agitation, on refroidit le mélange réactionnel à -5°C et on ajoute 16,1 g de triacétoxyborohydrure de sodium. Le mélange réactionnel est agité 5 heures entre -5°C et 0°C puis on laisse remonter la température à environ 20°C en 12 heures. On ajoute alors 11,35 g de paraformaldéhyde, puis après 6 heures d'agitation, 16,1 g de triacétoxyborohydrure de sodium. On agite alors 1 heure avant addition de 2,27 g de paraformaldéhyde. Après 16 heures d'agitation, le mélange réactionnel est filtré sur Célite. La Célite est lavée par 300 cm³ de méthanol, le filtrat est concentré à sec sous pression réduite (2,7 kPa), pour donner un résidu qui est diluée dans 500 cm³ de dichlorométhane. La phase organique est lavée par 600 cm³ d'une solution aqueuse à 5% en bicarbonate de sodium. La phase organique est décantée et la phase aqueuse est reprise par 2 fois 500 cm³ de dichlorométhane. Les phases organiques sont réunies, séchées sur sulfate de magnésium, filtrées puis concentrées à sec sous pression réduite (2,7 kPa) pour donner 23 g d'une poudre marron qui est purifiée par chromatographie-flash [éluant : dichlorométhane-méthanol-acétonitrile (90-5-5 en volumes)]. On obtient ainsi 3,4 g de (16R)-16-diméthylamino-16-désoxopristinamycine II_{B} sous forme d'une poudre beige-marron fondant vers 122°C (déc.).

Spectre de R.M.N. ¹ H (400 MHz, CDCl₃, δ en ppm) : 0,95 et 0,98 (2 d, J = 6,5 Hz, 3H chacun : CH₃ en 30 et CH₃ en 31) ; 1,07 (d, J = 6,5 Hz, 3H : CH₃ en 32) ; de 1,60 à 2,00 (mt, 6H : CH₂ en 15 - CH₂ en 25 - 1H du CH₂ en 26 et CH en 29) ; 1,78 (s, 3H : CH₃ en 33) ; 2,11 (mt, 1H : 1H du CH₂ en 26) ; 2,36 (s, 6H : N(CH₃)₂) ; 2,61 (dd, J = 16 et 10 Hz, 1H : 1H du CH₂ en 17) ; 2,72 (mt, 1H : CH en 4) ; 2,98 (dd, J = 16 et 4 Hz, 1H : 1H du CH₂ en 17) ; 3,21 (mt, 1H : CH en 16) ; 3,52 (mt, 1H : 1H du CH₂ en 9) ; 3,83 et 3,92 (2 mts, 1H chacun : CH₂ en 24) ; 4,32 (mt, 1H : 1H du CH₂ en 9) ; 4,67 (mt, 1H : CH en 14) ; 4,74 (dd, J = 9 et 3 Hz, 1H : CH en 27) ; 4,79 (dd, J = 10 et 2 Hz, 1H : CH en 3) ; 5,35 (d, J = 9 Hz, 1H : CH en 13) ; 5,65 (mt, 1H : CH en 10) ; 5.78 (dd, J = 16 et 2 Hz, 1H : CH en 6) ; 6,07 (mt ,1H : CONH) ; 6,17 (d, J = 16 Hz, 1H : CH en 11) ; 6,53 (dd, J = 16 et 5 Hz, 1H : CH en 5) ; 8,06 (s : CH en 20).

### Exemple 11

### (16R)-16-(Allyl)(méthyl)amino-16-désoxopristinamycine II_{B}

A 7 g de pristinamycine II_{B} en solution dans 100 cm³ de méthanol, on ajoute à environ 20°C, sous atmosphère d'azote, 20 g de sulfate de magnésium et 2,3 cm³ d'allylamine. Après 21 heures 45 minutes d'agitation, on ajoute 0,5 cm³ d'allylamine puis après 4 heures 45 minutes, 1,67 g de cyanoborohydrure de sodium puis 7 cm³ d'acide acétique. Le mélange réactionnel est agité 4 heures 30 minutes avant ajout de 100 mg de cyanoborohydrure de sodium supplémentaires. Après 25 heures d'agitation supplémentaires, 2,39 g de paraformaldéhyde sont additionnés et l'agitation est poursuivie. La même quantité de paraformaldéhyde est ajoutée 3 fois à une demi-heure d'intervalle et une heure après la dernière addition, on ajoute 450 mg de cyanoborohydrure de sodium, 3,5 cm³ d'acide acétique puis à nouveau 2,39 g de paraformaldéhyde. Après 19 heures 30 minutes d'agitation, le mélange est filtré sur Célite puis rinçé au méthanol. Le filtrat est concentré à sec sous pression réduite (2,7kPa) à 30°C puis le résidu obtenu repris par 300 cm³ de chlorure de méthylène et 600 cm³ d'une solution de bicarbonate de sodium à 5%. La phase aqueuse est décantée puis extraite par 200 cm³ de chlorure de méthylène. Les phases organiques sont rassemblées, lavées par 300 cm³ d'eau distillée, séchées sur sulfate de magnésium, filtrées puis concentrées à sec, sous pression réduite (2,7 kPa ), pour donner un solide qui est séché sous pression réduite (90 Pa), à 20°C, puis purifié par chromatographie flash [éluant : dichlorométhane-méthanol (95-5 en volumes)]. On obtient 1,25 g de (16R)-16-(allyl)(méthyl)amino-16-désoxopristinamycine II_{B}, sous forme d'un solide blanc cassé fondant vers 124°C (déc.).

Spectre de R.M.N. ¹ H (400 MHz, CDCl₃, δ en ppm) : 0,95 et 0,99 (2 d, J = 6,5 Hz, 3H chacun : CH₃ en 30 et CH₃ en 31) ; 1,08 (d, J = 6,5 Hz, 3H : CH₃ en 32) ; de 1,65 à 2,00 (mt, 6H : CH₂ en 15 - CH₂ en 25 - 1H du CH₂ en 26 et CH en 29) ; 1,78 (s, 3H : CH₃ en 33) ; 2,12 (mt, 1H : 1H du CH₂ en 26) ; 2,32 (s, 3H : NCH₃) ; 2,64 et 2,98 (2 dd, respectivement J = 16 et 10 Hz et J = 16 et 4 Hz, 1H chacun : CH₂ en 17) ; 2,73 (mt, 1H : CH en 4) ; 3,05 et 3,28 (2 dd, respectivement J = 14 et 7 Hz et J = 14 et 6 Hz, 1H chacun : CH₂ de N(Allyle)) ; 3,35 (mt, 1H : CH en 16) ; 3,52 (mt, 1H : 1H du CH₂ en 9) ; 3,84 et 3,93 (2 mts, 1H chacun : CH₂ en 24) ; 4,34 (mt, 1H : 1H du CH₂ en 9) ; 4,67 (mt, 1H : CH en 14) ; 4,75 (dd, J = 9 et 2,5 Hz, 1H : CH en 27) ; 4,80 (dd, J = 8 et 1,5 Hz, 1H : CH en 3) ; 5,19 et 5,23 (respectivement d, J = 10 Hz et dd, J = 18 et 1,5 Hz, 1H chacun : =CH₂ de l'allyle) ; 5,36 (d, J = 9 Hz, 1H : CH en 13) ; 5,67 (mt, 1H : CH en 10) ; 5,80 (dd, J = 17 et 2,5 Hz, 1H : CH en 6) ; de 5,80 à 5,95 (mt, 1H : CH= de l'allyle) ; 6,02 (mt, 1H : CONH) ; 6,18 (d, J = 16 Hz, 1H : CH en 11) ; 6,53 (dd, J = 17 et 5 Hz, 1H : CH en 5) ; 8,07 (s, 1H : CH en 20).

### Exemple 12

### 16-Propyne-2-ylamino-16-désoxopristinamycine II_{A}

### [mélange des isomères (16R)/(16S)=65/35]

A 0,5 g de pristinamycine II_{A} en solution dans 70 cm³ d'acétonitrile anhydre, on ajoute à une température voisine de 20°C, sous atmosphère d'argon, 0,130 cm³ de propargylamine puis 0,053 cm³ d'acide acétique. Le mélange est agité 18 heures à une température voisine de 20°C puis on ajoute 0,13 cm³ de propargylamine supplémentaire. Le mélange est agité 4 heures à une température de 20°C puis est concentré, sous pression réduite (2.7 kPa), à une température voisine de 30°C. jusqu'à apparition d'un insoluble. On additionne ensuite à une température voisine de 20°C sous atmosphère d'argon, 0,072 g de cyanoborohydrure de sodium puis 1.2 cm³ d'acide acétique concentré. Le mélange est agité 1 heure 30 minutes, à une température voisine de 20°C. Le mélange réactionnel est concentré à sec, sous pression réduite (2,7 kPa). Le résidu obtenu est ensuite repris par du dichlorométhane et la phase organique lavée deux fois avec une solution aqueuse saturée en bicarbonate de sodium. Les phases aqueuses sont réunies et extraites par du dichlorométhane. Les phases organiques sont réunies, séchées sur sulfate de magnésium, filtrées sur verre fritté puis concentrées à sec, sous pression réduite (2,7 kPa). Le résidu obtenu est purifié par chromatographie sur plaque préparative (gel de silice 60F₂₅₄ Merck; épaisseur=2mm, 20x20 cm), en éluant avec un mélange dichlorométhane-méthanol-acétonitrile (90-5-5 en volumes) pour donner 0,205 g de 16-Propyne-2-ylamino-16-désoxopristinamycine II_{A} (mélange des isomères (16R)/(16S)=65/35), sous forme d'une poudre beige.

Spectre de R.M.N. ¹ H [isomère Syn (16R) 65 % et isomère Anti (16S) 35 %] (400 MHz, CDCl₃, δ en ppm) : de 0,90 à 1,05 (mt, 6H : CH₃ en 30 et CH₃ en 31 ; de 1,05 à 1,20 (mt, 3H : CH₃ en 32) ; de 1,50 à 2,10 (mt : CH₂ en 15 et CH en 29) ; 1,64 et 1,74 (2 s : respectivement CH₃ en 33 de l'isomère Anti et CH₃ en 33 de l'isomère Syn) ; 2,27 et 2,32 (2 t, J = 2,5 Hz : respectivement CH du 2-propynyl de l'isomère Syn et CH du 2-propynyl de l'isomère Anti ) ; de 2,55 à 2,95 (mt : CH₂ en 25 - 1H du CH₂ en 17 - CH en 16 de l'isomère Syn et CH en 4) ; 3,00 (dd, J = 14 et 2,5 Hz : 1H du CH₂ en 17 de l'isomère Syn) ; 3,22 (dd, J = 14 et 2,5 Hz : 1H du CH₂ en 17 de l'isomère Anti ) ; 3,36 (mt : 1H du CH₂ en 9 de l'isomère Anti ) ; de 3,45 à 3,60 (mt : NCH₂ du 2-propynyl et CH en 16 de l'isomère Anti ) ; 3,82 (d large, J = 18 Hz : 1H du CH₂ en 9 de l'isomère Syn) ; de 4,10 à 4,60 (mt : CH₂ en 24 - 1H du CH₂ en 9 et CH en 14 de l'isomère Syn) ; de 4,75 à 4,85 (mt : CH en 14 de l'isomère Anti et CH en 13 de l'isomère Syn) ; de 4,90 à 5,00 (mt, 1H : CH en 3) ; de 5,45 à 5,60 (mt : CH en 10 de l'isomère Anti ) ; 5.50 (d, J = 8 Hz : CH en 13 de l'isomère Anti ) ; 5,64 (mt : CH en 10 de l'isomère Syn) ; de 5,80 à 6,10 (mt : CH en 6 - CH en 11 et CH en 26 de l'isomère Anti ) ; 6,13 (t, J = 3 Hz : CH en 26 de l'isomère Syn) ; 6,53 (dd, J = 16 et 6 Hz : CH en 5 de l'isomère Anti ) ; de 6,55 à 6,70 (mt : CONH de l'isomère Anti ) ; 6,61 (dd, J = 16 et 7 Hz : CH en 5 de l'isomère Syn) ; 7,48 (mt : CONH de l'isomère Syn) ; 7,87 et 8,08 (2 s : respectivement CH en 20 de l'isomère Syn et CH en 20 de l'isomère Anti ).

### Exemple 13

### 16-Allylamino-16-désoxopristinamycine II_{A}

### [mélange des isomères (16R)/(16S) = 65/35]

A une suspension de 0,5 g de pristinamycine II_{A} dans 15 cm³ d'acétonitrile et 0,143 cm³ d'atlylamine, maintenue à une température voisine de 20°C, on ajoute 0,054 cm³ d'acide acétique. Après une heure à une température voisine de 20°C, on ajoute successivement 0,072 g de cyanoborohydrure de sodium puis 1 cm³ d'acide acétique. Après une heure à une température voisine de 20°C, on ajoute au mélange réactionnel 7 cm³ d'eau et 15 cm³ de dichlorométhane. La phase organique est décantée puis lavée avec deux fois 10 cm³ d'une solution saturée en bicarbonate de sodium. Les phases aqueuses sont extraites avec 10 cm³ de dichlorométhane. Les phases organiques rassemblées sont séchées sur sulfate de magnésium, filtrées, concentrées sous pression réduite (environ 2,7 kPa) à une température voisine de 40°C. On obtient ainsi une meringue jaune vif que l'on purifie par chromatographie préparative sur couche mince : 6 plaques préparatives Merck, Kieselgel 60F254. 20x20 cm, épaisseur 2 mm, dépôt en solution dans le dichlorométhane, en éluant par un mélange dichlorométhane-méthanol-acétonitrile (80-10-10 en volumes) pour donner 0,179 g de 16-Allylamino-16-désoxopristinamycine II_{A} (mélange des isomères (16R):(16S) = 65/35), sous forme d'une meringue crème.

Spectre de R.M.N. ¹ H [mélange des isomères (16R):(16S) = 65/35] (400 MHz, CDCl₃, δ en ppm) : de 0,95 à 1,05 (mt, 6H : CH₃ en 30 et CH₃ en 31) ; de 1,10 à 1,20 (mt, 3H : CH₃ en 32) ; 1,64 et 1,71 (2 s, 3H en totalité : respectivement CH₃ en 33 de l'isomère Anti et CH₃ en 33 de l'isomère Syn) ; de 1,70 à 2,10 (mt, 3H : CH₂ en 15 et CH en 29) ; de 2,60 à 2,90 (mt : CH₂ en 25 - 1H du CH₂ en 17 - CH en 16 de l'isomère Syn et CH en 4) ; 3,06 (d large, J = 14 Hz : 1H du CH₂ en 17 de l'isomère Syn) ; 3,22 (d large, J = 15 Hz : 1H du CH₂ en 17 de l'isomère Anti ) ; de 3,30 à 3,55 (mt : 1H du CH₂ en 9 de l'isomère Anti - NCH₂ de l'Allyle et CH en 16 de l'isomère Anti ) ; 3,85 (d large, J = 18 Hz : 1H du CH₂ en 9 de l'isomère Syn) ; de 4,10 à 4,60 (mt : CH₂ en 24 - 1H du CH₂ en 9 et CH en 14 de l'isomère Syn) ; 4,80 (mt : CH en 14 de l'isomère Anti ) ; 4,87 (d large, J = 8 Hz : CH en 13 de l'isomère Syn) ; de 4,90 à 5,00 (mt, 1H : CH en 3) ; de 5,15 à 5,30 (mt : =CH₂ de l'Allyle) ; de 5,45 à 5,60 (mt : CH en 10 de l'isomère Anti ) ; 5,55 (d, J = 9 Hz : CH en 13 de l'isomère Anti ) ; 5,63 (mt : CH en 10 de l'isomère Syn) ; de 5,85 à 6,10 (mt : CH en 6 - CH en 11 - CH en 26 de l'isomère Anti et =CH de l'Allyle) ; 6,14 (s large : CH en 26 de l'isomère Syn) ; de 6,45 à 6,60 (mt : CH en 5 de l'isomère Anti et CONH de l'isomère Anti ) ; 6,62 (dd, J = 16 et 7 Hz : CH en 5 de l'isomère Syn) ; 7,39 (mt : CONH de l'isomère Syn) ; 7,88 et 8,07 (2 s : respectivement CH en 20 de l'isomère Syn et CH en 20 de l'isomère Anti ).

### Exemple 14

### (16R)-16-Diméthylamino-16-désoxopristinamycine II_{A}

A 28,5 g de pristinamycine II_{A} en solution dans 780 cm³ d'acétonitrile anhydre, on ajoute à une température voisine de 20°C, sous atmosphère d'argon, 6,9 cm³ de méthylamine (8 M dans l'éthanol) puis 1,43 cm³ d'acide acétique. On agite 48 heures à une température voisine de 20°C puis on additionne sous atmosphère d'argon, 3,8 g de cyanoborohydrure de sodium et 12 cm³ d'acide acétique. Le mélange est agité 3 heures à une température voisine de 20°C avant un nouvel ajout de 11 cm³ d'acide acétique. Le mélange réactionnel est encore agité 7,5 heures à une température voisine de 20°C. On ajoute alors 6 g de paraformaldéhyde et on laisse le mélange sous agitation durant 17 heures, à une température voisine de 20°C. La suspension blanche obtenue est filtrée et le filtrat est concentré sous pression réduite (2,7 kPa), à une température voisine de 30°C. L'huile épaisse résiduelle est ensuite reprise par 800 cm³ d'acétate d'éthyle et par 300 cm³ d'eau. Après agitation durant environ 15 minutes, le pH de la solution obtenue est amenée d'abord à 9 par addition de soude concentrée, puis à 11 par addition de 150 cm³ de soude 1N. Le mélange obtenu est agité une heure environ, avant un nouvel ajout de 50 cm³ de soude 1N et agitation durant encore une heure environ. Le mélange résultant est décanté et la phase organique lavée par 2 fois 100 cm³ d'eau puis extraite trois fois par du HCl 1N (successivement 1000 cm³, 100 cm³ et 50 cm³). Les phases aqueuses acides rassemblées sont extraites par 200 cm³ d'éther, puis alcalinisées à pH 10-11 par addition de 23 cm³ de soude concentrée. La phase aqueuse obtenue est extraite par 2 fois 300 cm³ de dichlorométhane et les phases organiques sont réunies, lavées par 100 cm³ d'eau, séchées sur sulfate de magnésium, filtrées sur verre fritté puis concentrées à sec, sous pression réduite (2,7 kPa), à une température voisine de 30°C pour donner un solide blanc. Celui-ci est agité dans 200 cm³ d'éther puis filtré et séché à poids constant (90 Pa, à environ 20°C), pour donner 20 g d'une poudre blanche. Celle-ci est purifiée par chromatographie flash [éluant : dichlorométhane-méthanol-acétonitrile (92-4-4 puis 84-8-8 en volumes) pour donner 5,2 g de (16R)-16-diméthylamino-16-désoxopristinamycine II_{A}, sous forme d'un solide blanc. 4,5 g de ce solide sont recristallisés dans un mélange acétonitrile-eau (18 cm³-9 cm³) pour donner, après essorage et séchage sous pression réduite (90 Pa, à environ 20°C), 3,46 g d'une poudre blanche fondant vers 212°C.

Spectre de R.M.N. ¹ H : (400 MHz, CDCl₃, δ en ppm) : de 0.90 à 1,05 (mt, 6H : CH₃ en 30 et CH₃ en 31) ; 1,13 (d, J = 6,5 Hz, 3H : CH₃ en 32) ; 1,63 (mt, 1H : 1H du CH₂ en 15) ; 1,73 (s, 3H : CH₃ en 33) ; de 1,95 à 2,10 (mt, 2N : 1H du CH₂ en 15 et CH en 29) ; 2,36 (s, 6H : N(CH₃)₂) ; de 2,50 à 2,65 (mt, 2H : 1H du CH₂ en 17 et CH en 16) ; de 2,65 à 2,75 (mt, 2H : CH en 4 et 1H du CH₂ en 25) ; de 2,80 à 2,95 (mt, 1H : 1H du CH₂ en 25) ; 2,97 (d, J = 11 Hz, 1H : 1H du CH₂ en 17) ; 3,79 (d large, J = 18 Hz, 1H : 1H du CH₂ en 9) ; 4,21 (mt, 1H : 1H du CH₂ en 24) ; de 4,30 à 4,50 (mt, 3H : 1H du CH₂ en 9 - 1H du CH₂ en 24 et CH en 14) ; 4,79 (d, J = 9 Hz, 1H : CH en 13) ; 4,97 (dd, J = 10 et 1,5 Hz, 1H : CH en 3) ; 5,64 (mt, 1H : CH en 10) ; 5,90 (d large, J = 16 Hz, 1H : CH en 11) ; 6,04 (d, J = 16 Hz, 1H : CH en 6) ; 6,13 (t, J = 3 Hz, 1H : CH en 26) ; 6,62 (dd, J = 17 et 5 Hz, 1H : CH en 5) ; 7,51 (mt, 1H : CONH) ; 7,87 (s : CH en 20).

### Exemple 15

### (16R)-16-Méthylamino-16-désoxopristinamycine II_{A}

En opérant comme décrit dans les exemples 12 et 13, à partir de pristinamycine IIA, on obtient la (16R)-16-méthylamino-16-désoxopristinamycine II_{A}, sous forme d'une meringue crème fondant vers 130 °C (déc.).

Spectre de R.M.N. ¹ H (400 MHz, CDCl₃, δ en ppm) : de 0,90 à 1,05 (mt, 6H : CH₃ en 30 et CH₃ en 31) ; 1,12 (d, J = 6,5 Hz, 3H : CH₃ en 32) ; 1,72 (s, 3H : CH₃ en 33) ; de 1,80 à 2,10 (mt, 3H : CH₂ en 15 et CH en 29) ; 2,58 (s, 3H : NCH₃) ; de 2,60 à 2,90 (mt, 4H : CH en 4 - 1H du CH₂ en 17 et CH₂ en 25) ; 3,12 (dd, J = 14 et 1,5 Hz, 1H : 1H du CH₂ en 17 ) ; de 3,35 à 3,45 (mt, 1H : CH en 16) ; 3,86 (d large, J = 18 Hz, 1H : 1H du CH₂ en 9) ; de 4,18 à 4,40 (mt, 3H : 1H du CH₂ en 9 et CH₂ en 24) ; 4,55 ( mt, 1H : CH en 14) ; 4,89 (d, J = 9 Hz, 1H : CH en 13) ; 4,93 (dd, J = 10 et 1,5 Hz, 1H : CH en 3) ; 5,62 (mt, 1H : CH en 10) ; 5,90 (d, J = 16 Hz, 1H : CH en 11) ; 6,01 (d, J = 16 Hz, 1H : CH en 6) ; 6,14 (t, J = 3 Hz, 1H : CH en 26) ; 6,61 (dd, J = 17 et 5 Hz, 1H : CH en 5) ; 7,38 (mt, 1H : CONH) ; 7,90 (s, 1H : CH en 20).

### Exemple 16

### 16-Benzylamino-16-désoxopristinamycine II_{A}

### [mélange des isomères (16R)/( 16S) = 50/50]

En opérant comme décrit dans les exemples 12 et 13, à partir de pristinamycine IIA, on obtient la 16-benzylamino-16-désoxopristinamycine II_{A} [mélange des isomères (16R)/(16S) = 50/50], sous forme d'une poudre blanche.

Spectre de R.M.N. ¹ H [ isomère Syn (16R) 50 % et isomère Anti (16S) 50 %] (400 MHz, CDCl₃, δ en ppm) : de 0,95 à 1,05 (mt, 6H : CH₃ en 30 et CH₃ en 31) ; de 1,05 à 1,20 (mt, 3H : CH₃ en 32) ; 1,45 et 1,63 (2 s : respectivement CH₃ en 33 de l'isomère Anti et CH₃ en 33 de l'isomère Syn) ; de 1,50 à 2,15 (mt, les 3H correspondant à : CH₂ en 15 et CH en 29) ; de 2,60 à 2,95 (mt : CH₂ en 25 - 1H du CH₂ en 17 de l'isomère Anti - CH₂ en 17 de l'isomère Syn et CH en 4) ; 3,12 (mt : CH en 16 de l'isomère Syn) ; 3,29 (d large, J = 15 Hz : 1H du CH₂ en 17 de l'isomère Anti ) ; 3,35 (mt : 1H du CH₂ en 9 de l'isomère Anti ) ; 3,47 (mt : CH en 16 de l'isomère Anti ) ; de 3,80 à 4,10 (mt : 1H du CH₂ en 9 de l'isomère Syn et NCH₂ du Benzyle) ; de 4,10 à 4,45 (mt : CH₂ en 24 - 1H du CH₂ en 9 de l'isomère Syn et CH en 14 de l'isomère Syn) ; 4,54 (mt : 1H du CH₂ en 9 de l'isomère Anti ) ; de 4,75 à 4,85 (mt : CH en 14 de l'isomère Anti et CH en 13 de l'isomère Syn) ; de 4,90 à 5,00 (mt, 1H : CH en 3) ; 5,40 (d, J = 8 Hz : CH en 13 de l'isomère Anti ) ; de 5,45 à 5,65 (mt, 1H : CH en 10) ; de 5,80 à 6,05 (mt, 2H : CH en 6 et CH en 11) ; 6,08 et 6,14 (2 t, J = 3 Hz, 1 H en totalité : respectivement CH en 26 de l'isomère Anti et CH en 26 de l'isomère Syn) ; de 6,45 à 6,55 (mt : CONH de l'isomère Anti ) ; 6,54 (dd, J = 16 et 6 Hz : CH en 5 de l'isomère Anti ) ; 6,61 (dd, J = 16 et 7 Hz : CH en 5 de l'isomère Syn) ; de 7,25 à 7,45 (mt : 5H du Phényle et CONH de l'isomère Syn) ; 7,86 et 8,09 (2 s : respectivement CH en 20 de l'isomère Syn et CH en 20 de l'isomère Anti ).

### Exemple 17

### (16R)-16-Méthoxyamino-16-désoxopristinamycine II_{B}

On place dans un ballon maintenu sous atmosphère d'azote, 10g de O-méthyloxime de la pristinamycine II_{B} (mélange 70 /30 des isomères Z et E), en solution dans 300 cm³ de méthanol et 100 cm³ d'acide acétique. Le mélange est refroidi à -70°C avant ajout de 10,3g de cyanoborohydrure de sodium. La température est laissée lentement remontée à environ 20°C et la réaction laissée sans agitation 48 heures. Un courant d'argon est passé pendant une heure dans la solution maintenue sous hotte aspirante, puis les solvants sont évaporés sous pression réduite (2,7kPa) à 20°C et le résidu repris par 200 cm³ de chlorure de méthylène et 100 cm³ d'eau distillée. La phase aqueuse est alcalinisée à pH 8 par addition de 30 cm³ de NaOH concentrée et le mélange agité 30 minutes avant d'être transféré en ampoule à décanter. La phase organique est décantée puis lavée par 2 fois 100 cm³ d'eau distillée. La phase organique est décantée, séchée sur sulfate de magnésium, filtrée puis concentrée sous pression réduite (2,7kPa), à 20°C, pour donner une huile incolore qui est agitée dans l'éther diéthylique, le précipité obtenu est filtrée. On obtient ainsi 9,1 g d'une poudre blanche qui est purifiée par chromatographie-flash (éluant CH₂Cl₂-MeOH 97-3 en volumes). On isole 1,88 g de (16R)-16-méthoxyamino-16-désoxopristinamycine II_{B} sous forme d'une poudre blanche fondant à 195°C.

Spectre de R.M.N. ¹ H (600 MHz, CDCl₃, δ en ppm) : 0,95 et 1,00 (2 d, J = 6,5 Hz, 3H chacun : CH₃ en 30 et CH₃ en 31) ; 1,07 (d, J = 6,5 Hz, 3H : CH₃ en 32) ; de 1,60 à 2,00 (mt, 6H : CH₂ en 15 - CH, en 25 - 1H du CH₂ en 26 et CH en 29) ; 1,80 (s, 3H : CH₃ en 33) ; 2,15 (mt, 1H : 1H du CH₂ en 26) ; de 2,70 à 2,80 (mt, 1H : CH en 4) ; 2,76 et 3,24 (2 dd, respectivement J = 16 et 8 Hz et J = 16 et 4 Hz, 1H chacun : CH₂ en 17) ; de 3,45 à 3,55 (mt, 2H : CH en 16 et 1H du CH₂ en 9) ; 3,60 (s, 3H ; O CH₃) ; 3,92 (mt, 2H : CH₂ en 24) ; 4,43 (mt, 1H : 1H du CH₂ en 9) ; de 4,70 à 4,80 (mt, 3H : CH en 3 - CH en 14 et CH en 27) ; 5,34 (d, J = 9 Hz, 1H : CH en 13) : 5,70 (mt, 1H : CH en 10) ; 5,80 (dd, J = 16 et 2 Hz, 1H : CH en 6) ; 6,14 (mt, 1H : CONH) ; 6,18 (d, J = 16 Hz, 1H : CH en 11) ; 6,51 (dd, J = 16 et 5 Hz, 1H : CH en 5) ; 8,09 (s, 1H ; CH en 20).

La O-méthyloxime (mélange 70 /30 des isomères Z et E) de la pristinamycine II_{B} peut être préparée de la manière suivante :

On place dans un tricol 20 g de pristinamycine II_{B} en solution dans 800 cm³ de pyridine anhydre puis on ajoute 4,2 g de chlorhydrate de méthoxyamine. Après 21 heures d'agitation, la pyridine est évaporée sous presion réduite (2,7 kPa), à 40°C, puis le résidu obtenu repris par 500 cm³ de chlorure de méthylène et 1 litre d'eau distillée. La phase organique est décantée, lavée par deux fois 1 litre d'eau distillée, séchée sur sulfate de sodium, filtrée puis concentrée à sec sous presion réduite (2,7 kPa), à 40°C, pour donner un résidu qui est agité dans 300 cm³ d'éther diéthylique. Le précipité est filtré, séché sous presion réduite (90 Pa) puis purifié par chromatographie-flash (éluant CH₂Cl₂-MeOH 95-5 en volumes). On obtient ainsi 16,9 g de O-méthyloxime de la pristinamycine II_{B} (mélange 70/30 des isomères Z et E) sous forme d'un solide blanc fondant vers 198-199°C (déc) et qui est utilisé tel quel pour les opérations suivantes.

Spectre de R.M.N. ¹ H (300 MHz, CDCl₃, δ en ppm) : de 0,90 à 1,10 (mt, 9H : CH₃ en 30 - CH₃ en 31 et CH₃ en 32) ; 1,73 et 1,74 (2 s, 3H en totalité : respectivement CH₃ en 33 de l'isomère E et CH₃ en 33 de l'isomère Z) ; de 1,75 à 2,35 (mt : CH₂ en 25 - CH₂ en 26 - CH en 29 et 1H du CH₂ en 15 de l'isomère E) ; 2,51 et 2,65 (2 dd, respectivement J = 17 et 6 Hz et J = 17 et 5 Hz : CH₂ en 15 de l'isomère Z) ; de 2,65 à 2,80 (mt, 1H : CH en 4) ; 3,00 (dd, J = 13 et 6 Hz : 1H du CH₂ en 15 de l'isomère E) ; 3,22 (d, J = 6 Hz : OH de l'isomère Z) ; de 3,30 à 3,45 (mt, 1H : 1H du CH₂ en 9) ; 3,58 et 3,68 (2 d, J = 15 Hz : CH₂ en 17 de l'isomère E) ; de 3,65 à 3,80 (mt, 1H : 1H du CH₂ en 24) ; 3,62 et 4,04 (2 d, J = 16,5 Hz : CH₂ en 17 de l'isomère Z) ; 3,92 et 3,94 (2 s, 3H en totalité : respectivement OCH₃ de l'isomère Z et OCH₃ de l'isomère E) ; de 3,95 à 4,20 (mt, 1H : 1H du CH₂ en 24) ; de 4,35 à 4,55 (mt, 1H : 1H du CH₂ en 9) ; de 4,60 à 4,80 (mt, 2H : CH en 3 et CH en 27) ; de 4,80 à 4,90 (mt : CH en 13 de l'isomère E et CH en 14 de l'isomère Z) ; 5,06 (mt : CH en 14 de l'isomère E) ; 5,57 (d, J = 9 Hz : CH en 13 de l'isomère Z) ; de 5,60 à 5,90 (mt, 2H : CH en 10 et CH en 6) ; 6,05 et 6,14 (2 d, J = 16 Hz, 1H en totalité : respectivement CH en 11 de l'isomère E et CH en 11 de l'isomère Z) ; 6,28 (mt : CONH de l'isomère Z) ; 6,47 (d, J = 16 et 5 Hz, 1H : CH en 5) ; 7,47 (mt : CONH de l'isomère E) ; 7,77 (s : CH en 20 de l'isomère E) ; 8,08 (s : CH en 20 de l'isomère Z).

### Exemple 18

### (16R)-16-Ethoxyamino-16-désoxopristinamycine II_{B}

En opérant comme à l'exemple 17, mais à partir de 1,53 g de O-éthyloxime de la pristinamycine II_{B} (mélange 50 /50 des isomères Z et E) en solution dans 45 cm³ de méthanol , 15 cm³ d'acide acétique et 1,67 g de cyanoborohydrure de sodium et après 67 heures de réaction, on obtient 1,4 g d'un solide blanc qui est purifié par chromatographie-flash (éluant CH₂Cl₂-MeOH 97/3 en volume) pour donner 360 mg de (16R)-16-éthoxyamino-16-désoxopristinamycine II_{B} sous forme d'un solide blanc fondant à 205°C.

Spectre de R.M.N. ¹ H (400 MHz, CDCl₃, δ en ppm) : 0,96 et 1,00 (2 d, J = 6,5 Hz, 3H chacun : CH₃ en 30 et CH₃ en 31) ; 1,08 (d, J = 6,5 Hz, 3H : CH₃ en 32) : 1,19 (t, J = 7 Hz, 3H : CH₃ de l'éthyle) ; de 1,60 à 2,00 (mt, 6H : CH₂ en 15 - CH₂ en 25 - 1H du CH₂ en 26 et CH en 29) ; 1.80 (s, 3H : CH₃ en 33) ; 2,14 (mt, 1H : 1H du CH₂ en 26) ; de 2,70 à 2,80 (mt, 1H : CH en 4) ; 2,76 et 3,26 (2 dd, respectivement J = 16 et 8 Hz et J = 16 et 4 Hz, 1H chacun : CH₂ en 17) ; de 3,45 à 3,55 (mt, 2H : CH en 16 et 1H du CH₂ en 9) ; 3,79 (q, J = 7 Hz, 2H : CH₂ de l'éthyle) ; 3,93 (mt, 2H : CH₂ en 24) ; 4,43 (mt, 1H : 1H du CH₂ en 9) ; de 4,70 à 4,80 (mt, 3H : CH en 3 - CH en 14 et CH en 27) ; 5,34 (d, J = 9 Hz, 1H : CH en 13) ; 5,70 (mt, 1H : CH en 10) ; 5,80 (dd, J = 16 et 2 Hz, 1H : CH en 6) ; 6,13 (mt , 1H : CONH) ; 6,17 (d, J = 16 Hz, 1H : CH en 11) ; 6,51 (dd, J = 16 et 5 Hz, 1H : CH en 5) ; 8,08 (s, 1H : CH en 20).

La O-éthyloxime (mélange 70 /30 des isomères Z et E) de la pristinamycine II_{B} peut être préparée en opérant comme à l'exemple 17 mais à partir de 12 g de pristinamycine II_{B}, 2,44 g de chlorhydrate de O-éthyl hydroxylamine dans 400 cm³ de pyridine. Après extraction, et agitation dans l'éther diéthylique on obtient 11,28 g de O-éthyloxime de la pristinamycine II_{B} (mélange 70/30 des isomères Z et E), sous forme d'un solide jaune clair fondant vers 114°C (déc.) et qui est utilisé tel quel pour les opérations suivantes.

Spectre de R.M.N. ¹ H du mélange 70/30 des deux isomères Z/E (400 MHz, CDCl₃, δ en ppm) : de 0,90 à 1,10 (mt, 9H : CH₃ en 30 - CH₃ en 31 et CH₃ en 32) ; de 1,25 à 1,35 (mt, 3H : CH₃ de l'Ethyle) ; 1,70 et 1,75 (2 s, 3H en totalité : respectivement CH₃ en 33 de l'isomère E et CH₃ en 33 de l'isomère Z) ; de 1,75 à 2,35 (mt : CH₂ en 25 - CH₂ en 26 - CH en 29 et 1H du CH₂ en 15 de l'isomère E) ; 2,52 et 2,68 (2 dd, respectivement J = 16,5 et 6 Hz et J = 16,5 et 5 Hz : CH₂ en 15 de l'isomère Z) ; de 2,70 à 2.80 (mt, 1H : CH en 4) ; 3,02 (dd, J = 13 et 5 Hz : 1H du CH₂ en 15 de l'isomère E) ; 3,25 (d, J = 6 Hz : OH de l'isomère Z) ; de 3,30 à 3,45 (mt, 1H : 1H du CH₂ en 9) ; 3,61 et 3,72 (2 d, J = 15 Hz : CH₂ en 17 de l'isomère E) ; de 3,70 à 3,80 (mt, 1H : 1H du CH₂ en 24) ; 3,63 et 4,07 (2 d, J = 16 Hz : CH₂ en 17 de l'isomère Z) ; de 4,00 à 4,25 (mt, 3H : 1H du CH₂ en 24 et OCH₂) ; de 4,40 à 4,55 (mt, 1H : 1H du CH₂ en 9) ; de 4,65 à 4,90 (mt : CH en 27 - CH en 3 et CH en 14 de l'isomère Z) ; 4,91 (d, J = 9 Hz : CH en 13 de l'isomère E) ; 5,08 (mt : CH en 14 de l'isomère E) ; 5,59 (d, J = 9 Hz : CH en 13 de l'isomère Z) : de 5,65 à 5,80 (mt, 1H : CH en 10) ; 5,79 et 5,85 (2 dd, respectivement J = 17 et 2 Hz et J = 17 et 1,5 Hz, 1H en totalité : CH en 6 de l'isomère Z et CH en 6 de l'isomère E) ; 6,06 et 6,15 (2 d, J = 16 Hz. 1H en totalité : respectivement CH en 11 de l'isomère E et CH en 11 de l'isomère Z) ; 6,24 (mt : CONH de l'isomère Z) ; de 6,40 à 6,55 (mt, 1H : CH en 5) ; 7,43 (mt : CONH de l'isomère E) ; 7,79 (s : CH en 20 de l'isomère E) ; 8,09 (s : CH en 20 de l'isomère Z).

### Exemple 19

### (16R)-16-Allyloxyamino-16-désoxopristinamycine II_{B}

En opérant comme à l'exemple 17, mais à partir de 1,46 g de O-allyloxime de la pristinamycine II_{B} (mélange des isomères Z/E 65/35), en solution dans 42 cm³ de méthanol , 14 cm³ d'acide acétique et 1,57 g de cyanoborohydrure de sodium et après 96 heures de réaction, on isole 1,3 g d'un solide blanc qui est purifié par chromatographie flash (éluant CH₂Cl₂-MeOH 97/3 en volumes) pour donner 0.31 g de (16R)-16-allyloxyamino-16-désoxopristinamycine II_{B}, sous forme d'un solide blanc fondant à 130°C.

Spectre de R.M.N. ¹ H (400 MHz, CDCl₃, δ en ppm) : 0,95 et 1,00 (2 d, J = 6,5 Hz, 3H chacun : CH₃ en 30 et CH₃ en 31) : 1,07 (d, J = 6,5 Hz, 3H : CH₃ en 32) ; de 1,60 à 2,05 (mt, 6H : CH₂ en 15 - CH₂ en 25 - 1H du CH₂ en 26 et CH en 29) ; 1,80 (s, 3H : CH₃ en 33) ; 2,14 (mt, 1H : 1H du CH₂ en 26) : 2,24 (s large, 1H : OH) ; de 2,70 à 2,85 (mt, 1H : CH en 4) ; 2,77 et 3,27 (2 dd, respectivement J = 16 et 8 Hz et J = 16 et 4 Hz, 1H chacun : CH₂ en 17) ; de 3,45 à 3,55 (mt, 2H : CH en 16 et 1H du CH₂ en 9) ; 3,92 (mt, 2H : CH₂ en 24) ; 4,25 (mt, 2H : CH₂O) ; 4,43 (mt, 1H : 1H du CH₂ en 9) ; de 4,70 à 4,80 (mt, 3H : CH en 3 - CH en 14 et CH en 27) ; 5,23 et 5,30 (2 dd, respectivement J =10 et 1,5 Hz et J = 18 et 1,5 Hz, 1H chacun : =CH₂ de l'allyle) ; 5,34 (d, J = 9 Hz, 1H : CH en 13) ; 5,60 (mf, 1H : NH) ; 5,70 (mt, 1H : CH en 10) ; 5,80 (dd, J = 16 et 2 Hz, 1H : CH en 6) ; 5,95 (mt, 1H : CH de l'allyle) ; 6,12 (mt, 1H : CONH) ; 6,18 (d, J = 16 Hz, 1H : CH en 11) ; 6,51 (dd, J = 16 et 5 Hz, 1H : CH en 5) ; 8,09 (s, 1H : CH en 20).

La O-aliyloxime de la pristinamycine II_{B} (mélange 65/55 des isomères Z et E) peut être préparée en opérant comme à l'exemple 17, mais à partir de 5 g de pristinamycine II_{B}, 1,14 g de chlorhydrate de O-allyl hydroxylamine dans 200 cm³ de pyridine. Après extraction, et agitation dans l'éther d'éthylique, on obtient 4,2 g de O-allyloxime de la pristinamycine II_{B} (mélange 65/55 des isomères Z et E), sous forme d'un solide ocre fondant à 102-104°C et qui est utilisé tel quel pour les opérations suivantes.

Spectre de R.M.N. ¹ H (300 MHz, CDCl₃, δ en ppm) : de 0,90 à 1,10 (mt, 9H : CH₃ en 30 - CH₃ en 31 et CH₃ en 32) ; 1,73 et 1,74 (2 s, 3H en totalité : respectivement CH₃ en 33 de l'isomère E et CH₃ en 33 de l'isomère Z) ; de 1,75 à 2,35 (mt : CH₂ en 25 - CH₂ en 26 - CH en 29 et 1H du CH₂ en 15 de l'isomère E) ; 2,51 et 2,65 (2 dd, respectivement J = 17 et 6 Hz et J = 17 et 5 Hz : CH₂ en 15 de l'isomère Z) ; de 2,65 à 2,80 (mt, 1H : CH en 4) ; 3.00 (dd, J = 13 et 6 Hz : 1H du CH₂ en 15 de l'isomère E) ; 3,22 (d, J = 6 Hz : OH de l'isomère Z) ; de 3,30 à 3,45 (mt, 1H : 1H du CH₂ en 9) ; 3,58 et 3,68 (2 d, J = 15 Hz : CH₂ en 17 de l'isomère E) ; de 3,65 à 3,80 (mt, 1H : 1H du CH₂ en 24) ; 3,62 et 4,04 (2 d, J = 16,5 Hz : CH₂ en 17 de l'isomère Z) ; 3,92 et 3,94 (2 s, 3H en totalité : respectivement OCH₃ de l'isomère Z et OCH₃ de l'isomère E) ; de 3,95 à 4,20 (mt, 1H : 1H du CH₂ en 24) ; de 4,35 à 4,55 (mt, 1H : 1H du CH₂ en 9) ; de 4,60 à 4,80 (mt, 2H : CH en 3 et CH en 27) ; de 4,80 à 4,90 (mt : CH en 13 de l'isomère E et CH en 14 de l'isomère Z) ; 5,06 (mt : CH en 14 de l'isomère E) ; 5,57 (d, J = 9 Hz : CH en 13 de l'isomére Z) ; de 5,60 à 5,90 (mt, 2H : CH en 10 et CH en 6) ; 6,05 et 6,14 (2 d, J = 16 Hz, 1H en totalité : respectivement CH en 11 de l'isomère E et CH en 11 de l'isomère Z) ; 6,28 (mt : CONH de l'isomère Z) ; 6,47 (d, J = 16 et 5 Hz, 1H : CH en 5) ; 7,47 (mt : CONH de l'isomère E) ; 7,77 (s : CH en 20 de l'isomère E) ; 8,08 (s : CH en 20 de l'isomère Z).

### Exemple 20

### (16R)-16-Propyloxyamino-16-désoxopristinamycine II_{B}

En opérant comme à l'exemple 17, mais à partir de 1,5 g de O-propyloxime de la pristinamycine II_{B} (mélange 50/50 des isomère Z et E), en solution dans 45 cm³ de méthanol, 15 cm³ d'acide acétique et 1,61 g de cyanoborohydrure de sodium et après 50 heures de réaction, on isole 1,4 g d'un solide blanc qui est purifié par chromatographie flash (éluant CH₂Cl₂-MeOH 97/3 en volumes) pour donner 0,31 g de (16R)-16-propyloxyamino-16-désoxopristinamycine II_{B.} sous forme d'un solide blanc fondant à 135°C.

Spectre de R.M.N. ¹ H (400 MHz, CDCl₃, δ en ppm) : de 0,90 à 1,05 (mt, 9H : CH₃ en 30 - CH₃ en 31 et CH₃ du Propyle) ; 1,08 (d, J = 6,5 Hz, 3H : CH₃ en 32) ; de 1,55 à 1,70 (mt, 3H : 1H du CH₂ en 15 et CH₂ central du Propyle) ; de 1,70 à 2,00 (mt, 5H : 1H du CH₂ en 15 - CH₂ en 25 - 1H du CH₂ en 26 et CH en 29) ; 1,80 (s, 3H : CH₃ en 33) ; 2,14 (mt, 1H : 1H du CH₂ en 26) ; de 2,20 à 2,35 (mf étalé, 1H : OH) ; de 2,70 à 2,80 (mt, 1H : CH en 4) ; 2,75 (dd, J = 16 et 8 Hz, 1H : 1H du CH₂ en 17) ; 3,27 (dd, J = 16 et 4 Hz, 1H : 1H du CH₂ en 17) ; de 3,40 à 3,55 (mt, 2H : CH en 16 et 1H du CH₂ en 9) ; 3,69 (t, J = 6,5 Hz, 2H : OCH₂) ; de 3,85 à 4,00 (mt, 2H : CH₂ en 24) ; 4,43 (mt, 1H : 1H du CH₂ en 9) ; de 4,65 à 4,80 (mt, 3H : CH en 3 - CH en 14 et CH en 27) ; 5,36 (d, J = 9 Hz, 1H : CH en 13) ; de 5,40 à 5,60 (mf étalé, 1H : NH) ; 5,70 (mt, 1H : CH en 10) ; 5,80 (dd, J = 16 et 1,5 Hz, 1H : CH en 6) ; 6,14 (mt, 1H : CONH) : 6,17 (d, J = 16 Hz, 1H : CH en 11) ; 6,51 (dd, J = 16 et 5 Hz, 1H : CH en 5) ; 8,08 (s, 1 H : CH en 20).

La O-propyloxime de la pristinamycine II_{B} (mélange 85/15 des isomère Z et E) peut être préparée en opérant comme à l'exemple 17, mais à partir de 4 g de pristinamycine II_{B}, de 2,6 g de chlorhydrate de O-propyl hydroxylamine dans 60 cm³ de pyridine. Après extraction, et séchage sous pression réduite (2,7 kPa), à 20°C, on obtient un solide qui est agité dans l'acétonitrile pour donner après filtration du précipité 2,75 g de O-propyloxime de la pristinamycine II_{B} (mélange 85/15 des isomère Z et E), sous forme d'un solide blanc fondant à 130-132°C et qui est utilisé tel quel pour les opérations suivantes.

Spectre de R.M.N. ¹ H (400 MHz, CDCl₃, δ en ppm) : de 0,90 à 1,10 (mt, 12H : CH₃ en 30 - CH₃ en 31 - CH₃ en 32 et CH₃ du Propyle) ; de 1,60 à 1,75 (mt, 2H : CH₂ central du Propyle) ; 1,75 (s. 3H : CH₃ en 33) ; de 1,75 à 2,00 (mt, 4H : CH₂ en 25 - 1H du CH₂ en 26 et CH en 29) ; 2,15 (mt, 1H : 1H du CH₂ en 26) ; 2,51 et 2,65 (2 dd, respectivement J = 17 et 6 Hz et J = 17,5 et 5 Hz, 1H chacun : CH₂ en 15) ; 2,74 (mt, 1H : CH en 4) ; 3,20 (d, J = 6 Hz, 1H : OH) ; 3,38 (mt, 1H : 1H du CH₂ en 9) ; 3,65 (d, J = 15 Hz, 1H : 1H du CH₂ en 17) ; 3,74 (mt, 1H : 1H du CH₂ en 24) ; de 3,95 à 4,10 (mt, 4H : 1H du CH₂ en 24 - 1H du CH₂ en 17 et OCH₂) ; 4,43 (mt, 1H : 1H du CH₂ en 9) ; 4,67 (dd, J = 10 et 3 Hz, 1H : CH en 27) ; 4,72 (d large, J = 10 Hz, 1H : CH en 3) ; 4,83 (mt, 1H : CH en 14) ; 5.55 (d, J = 9 Hz, 1H : CH en 13) ; 5,69 (mt, 1H : CH en 10) ; 5,78 (dd, J = 17 et 1,5 Hz, 1H : CH en 6) ; 6,13 (d, J = 16 Hz, 1H : CH en 11) ; 6,26 (mt, 1H : CONH) ; 6,46 (dd, J = 17 et 5 Hz, 1H : CH en 5) ; 8,07 (s, 1H : CH en 20).

### Exemple 21

### (16R)-16-Méthoxyamino-16-désoxopristinamycine II_{A}

Ce composé peut être obtenue en opérant comme à l'exemple 17, mais à partir de 3 g de O-méthyloxime de la pristinamycine II_{A} (mélange 65/25 des isomère Z et E), en solution dans 90 cm³ de méthanol, 30 cm³ d'acide acétique et 3,4 g de cyanoborohydrure de sodium et après une semaine de réaction à environ 20°C et une semaine de réaction à 30-33°C. On obtient ainsi 3 g d'un solide blanc qui est purifié par chromatographie flash (éluant CH₂Cl₂ - MeOH 97/3 en volumes) pour donner 0,36 g de (16R)-16-méthoxyamino-16-désoxopristinamycine II_{A}, sous forme d'un solide blanc fondant à 150°C.

Spectre de R.M.N. ¹ H (400 MHz, CDCl₃, δ en ppm) : de 0,90 à 1,05 (mt, 6H : CH₃ en 30 et CH₃ en 31) ; 1,13 (d, J = 6,5 Hz, 3H : CH₃ en 32) ; 1,59 (s large, 1H : OH) ; de 1,65 à 1,85 (mt, 2H : CH₂ en 15) ; 1,73 (s, 3H : CH₃ en 33) ; 2,03 (mt, 1H : CH en 29) ; de 2,60 à 2,85 (mt, 2H : CH en 4 et 1H du CH₂ en 25) ; 2,64 (dd, J = 14 et 11 Hz, 1H : 1H du CH₂ en 17) ; 2,85 (mt, 1H : 1H du CH₂ en 25) ; 2,95 (mt, 1H : CH en 16) ; 3,20 (dd, J = 14 et 2,5 Hz, 1H : 1H du CH₂ en 17) ; 3,60 (s, 3H : OCH₃) ; 3,81 (d large, J = 18 Hz, 1H : 1H du CH₂ en 9) ; 4,22 (mt, 1H : 1H du CH₂ en 24) ; de 4,30 à 4,55 (mt, 3H : 1H du CH₂ en 9 - 1H du CH₂ en 24 et CH en 14) ; 4,83 (d, J = 9 Hz, 1H : CH en 13) ; 4,96 (d large, J = 10 Hz, 1H : CH en 3) ; 5,47 (s large, 1H ; NH) ; 5,65 (mt, 1H : CH en 10) ; 5,90 (d large J = 16 Hz, 1H : CH en 11) ; 6,03 (d large, J = 17 Hz, 1H : CH en 6) ; 6,14 (t, J = 3 Hz, 1H : CH en 26) ; 6,62 (dd, J = 17 et 7 Hz, 1H : CH en 5) ; 7,48 (mt, 1H : CONH) ; 7,87 (s, 1H : CH en 20).

La O-méthyloxime de la pristinamycine II_{A} (mélange 65/35 des isomère Z et E) peut être obtenue en opérant comme à l'exemple 17, mais à partir de 8 g de pristinamycine II_{A} et de 1,43 g de chlorhydrate de méthoxyamine dans 80 cm³ de pyridine. Après évaporation de la pyridine sous pression réduite (2,7 kPa), à 45°C, extraction, agitation du produit dans 300 cm³ d'éther diéthylique, filtration et lavage à l'éther diéthylique, on obtient, après séchage sous pression réduite (90 Pa), à 40°C, 7,51 g de O-méthyloxime de la pristinamycine IIA (mélange 65/35 des isomère Z et E), sous forme d'un solide blanc fondant vers 204°C et utilisé tel quel dans les opérations suivantes.

### Exemple 22

### (16R)-16-(1-Pyrrolidinyl)amino-16-désoxopristinamycine II_{B}

En opérant comme à l'exemple 17, mais à partir de 3 g de pristinamycine II_{B} en solution dans 30 cm³ de méthanol, de 9 g de sulfate de magnésium, de 1,6 cm³ de triéthylamine et de 1,4 g de chlorydrate de 1-aminopyrrolidine et après avoir ajouté après 18 heures d'agitation, 0,43 g de cyanoborohydrure de sodium et 1,5 cm³ d'acide acétique, le mélange réactionnel est agité 4 heures et conduit après traitement à 3,5 g d'une poudre jaune qui est purifiée par chromatographie-flash [éluant : dichlorométhane-méthanol-acétonitrile (90-5-5 en volumes)]. On obtient ainsi un solide qui est agité dans de l'éther éthylique, séparé par filtration pour donner 0,56 g de (16R)-16-(1-pyrrolidinyl)amino-16-désoxopristinamycine II_{B}. sous forme d'une poudre beige fondant vers 130°C.

Spectre de R.M.N. ¹ H (400 MHz, CDCl₃, δ en ppm) : 0,95 et 1,00 (2 d. J = 6,5 Hz, 3H chacun : CH₃ en 30 et CH₃ en 31) ; 1,06 (d, J = 6,5 Hz, 3H : CH₃ en 32) ; 1,53 (mt, 1H : 1H du CH₂ en 15) ; de 1,70 à 2,20 (mt, 10H : 1H du CH₂ en 15 - CH₂ en 25 - CH₂ en 26 - CH en 29 et 2 CH₂ de Pyrrolidinyle) ; 1,83 (s, 3H : CH₃ en 33) ; de 2,70 à 2,90 (mt, 6H : CH en 4 - 1H du CH₂ en 17 et 2 CH₂N de Pyrrolidinyle) ; 3,06 (dd, J = 16 et 4 Hz, 1H : 1H du CH₂ en 17) ; de 3,40 à 3,50 (mt, 2H : 1H du CH₂ en 9 et CH en 16) ; 3,82 et 3,97 (2 mts, 1H chacun : CH₂ en 24) ; 4,37 (mt, 1H : 1H du CH₂ en 9) ; 4,57 (mt, 1H : CH en 14) ; 4,73 (dd, J = 9 et 3 Hz, 1H : CH en 27) ; 4,77 (dd, J = 10 et 2 Hz, 1H : CH en 3) ; 5,41 (d, J = 9 Hz, 1H : CH en 13) ; 5,68 (mt, 1H : CH en 10) ; 5,78 (dd, J = 17 et 2 Hz, 1H : CH en 6) ; 6,01 (mt, 1H : CONH) ; 6,18 (d, J = 16 Hz, 1H : CH en 11) ; 6,50 (dd, J = 17 et 5 Hz, 1H : CH en 5) ; 8,10 (s, 1H : CH en 20).

### Exemple 23

### (16R)-16-Diméthylamino-16-désoxypristinamycine II_{F}

A 0,27 g de pristinamycine II_{F} en solution dans 7 cm³ d'acétonitrile anhydre, on ajoute à une température voisine de 20°C, sous atmosphère d'argon, 0,069 cm³ de méthylamine (8M dans l'éthanol) puis 0,015 cm³ d'acide acétique. On agite 17 heures à une température voisine de 20°C puis on additionne sous atmosphère d'argon, 0,038 g de cyanoborohydrure de sodium et 0,13 cm³ d'acide acétique. Le mélange est agité 3 heures, à une température voisine de 20°C avant une nouvelle addition de 0,1 cm³ d'acide acétique. Le mélange réactionnel est encore agité 7 heures à une température voisine de 20 °C. On ajoute alors 0,9 g de paraformaldéhyde et on laisse le milieu sous agitation durant 17 heures à une température voisine de 20°C. La suspension blanche obtenue est filtrée et le filtrat concentré à sec sous pression réduite (2,7kPa), à une température voisine de 30°C. L'huile épaisse résiduelle est ensuite reprise par 15 cm³ d'acétate d'éthyle et par 3 cm³ d'eau. Après agitation pendant 15 minutes, le pH de la solution obtenue est amenée d'abord à 9 par addition de soude concentrée, puis à 11 par addition de 1,5 cm³ de soude 1N. Le mélange obtenu est agité une heure environ, décanté et la phase organique lavée par 2 fois 1 cm³ d'eau puis extraite trois fois par de l'acide chlorhydrique 1N (successivement 10 cm³, 1 cm³ et 0,5 cm³). Les phases aqueuses acides rassemblées sont lavées par 3 cm³ d'éther, puis alcalinisées à pH 10-11 par addition de soude concentrée. La phase aqueuse obtenue est extraite par 2 fois 4 cm³ de dichlorométhane et les phases organiques sont réunies, lavées par 2 cm³ d'eau, séchées sur sulfate de magnésium, filtrées sur verre fritté puis concentrées à sec, sous pression réduite (2,7 kPa), à une température voisine de 30°C pour donner un solide blanc. Celui-ci est agité dans 5 cm³ d'éther puis filtré et séché à poids constant (90 Pa à 20°C), pour donner 0,17 g d'une poudre blanche. Celle-ci est purifiée par chromatographie -flash [éluant : dichlorométhane-méthanol-acétonitrile (92-4-4) pour donner 0,067 g de (16R)-16-diméthylamino-16-désoxypristinamycine II_{F} sous forme d'un solide blanc fondant à 132°C.

Spectre de R.M.N. ¹H (400 MHz, CDCl₃, δ en ppm) : de 0,90 à 1,00 (mt, 6H : CH₃ en 30 et CH₃ de l'éthyle en 29) ; 1,07 (d, J = 6,5 Hz, 3H : CH₃ en 32) ; 1,18 et 1,50 (2 mts, 1H chacun : CH₂ de l'éthyle en 29) ; de 1,60 à 2,00 (mt, 6H : CH₂ en 15 - CH₂ en 25 - 1H du CH₂ en 26 et CH en 29) ; 1,78 (s, 3H : CH₃ en 33) ; 2,12 (mt, 1H : 1H du CH₂ en 26) ; 2,37 (s, 6H : N(CH₃)₂) ; 2,62 et 2,99 (2 dd, respectivement J = 16 et 10 Hz et J = 16 et 4 Hz, 1H chacun : CH₂ en 17) ; 2,76 (mt, 1H : CH en 4) ; 3,22 (mt, 1H : CH en 16) ; 3,51 (mt, 1H : 1H du CH₂ en 9) ; 3,83 et 3,94 (2 mts, 1H chacun : CH₂ en 24) ; 4,35 (mt, 1H : 1H du CH₂ en 9) ; 4,67 (mt, 1H : CH en 14) ; 4,75 (dd, J = 9 et 2,5 Hz. 1H : CH en 27) ; 4,91 (dd, J = 9,5 et 1,5 Hz, 1H : CH en 3) ; 5,35 (d, J = 9 Hz, 1H : CH en 13) ; 5,66 (mt, 1H : CH en 10) ; 5,80 (dd, J = 16 et 1,5 Hz, 1H : CH en 6) ; 6,02 (mt, 1H : CONH); 6,18 (d, J = 16 Hz, 1H : CH en 11) ; 6,54 (dd, J = 16 et 5 Hz, 1H : CH en 5) ; 8,07 (s, 1H : CH en 20).

### Exemple 24

En opérant par analogie avec les exemples ci-dessus, on prépare également les produits suivants :
- (16R)-16-(Méthoxy)(méthyl)amino-16-désoxopristinamycine II_{B}
- (16R)-16-(Méthoxy)(méthyl)amino-16-désoxopristinamycine II_{A}
- (16R)-16-(Ethoxy)(méthyl)amino-16-désoxopristinamycine II_{B}
- (16R)-16-(Ethoxy)(méthyl)amino-16-désoxopristinamycine II_{A}
- (16R)-16-(Méthyl)(propoxyl)amino-16-désoxopristinamycine II_{B}
- (16R)-16-(Méthyl)(propoxyl)amino-16-désoxopristinamycine II_{A}
- (16R)-16-(Allyloxy)(méthyl)amino-16-désoxopristinamycine II_{B}
- (16R)-16-(Allyloxy)(méthyl)amino-16-désoxopristinamycine II_{A}
- (16R)-16-(Cyclopropyl)(méthyl)amino-16-désoxopristinamycine II_{B}
- (16R)-16-(Cyclopropyl)(méthyl)amino-16-désoxopristinamycine II_{A}
- (16R)-16-(Méthyl)(propyne-2-yl)amino-16-désoxopristinamycine II_{B}
- (16R)-16-(Méthyl)(propyne-2-yl)amino-16-désoxopristinamycine II_{A}
- (16R)- 16-(Méthyl)(1-pyrrolidinyl)amino-16-désoxopristinamycine II_{B}

La présente invention concerne également les compositions pharmaceutiques contenant au moins un dérivé de streptogramine selon l'invention, à l'état pur, associé à un dérivé de streptogramine du groupe B, le cas échéant sous forme de sel, et/ou sous forme d'une association avec un ou plusieurs diluants ou adjuvants compatibles et pharmaceutiquement acceptables.

Les compositions selon l'invention peuvent être utilisées par voie orale, parentérale, topique, rectale ou en aérosols.

Comme compositions solides pour administration orale peuvent être utilisés des comprimés, des pilules, des gélules, des poudres ou des granulés. Dans ces compositions, le produit actif selon l'invention, généralement sous forme d'association est mélangé à un ou plusieurs diluants ou adjuvants inertes, tels que saccharose, lactose ou amidon. Ces compositions peuvent comprendre des substances autres que les diluants, par exemple un lubrifiant tel que le stéarate de magnésium ou un enrobage destiné à une libération contrôlée.

Comme compositions liquides pour administration orale, on peut utiliser des solutions pharmaceutiquement acceptables, des suspensions, des émulsions. des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration parentérale, peuvent être des solutions stériles ou des émulsions. Comme solvant ou véhicule, on peut employer le propylèneglycol, un polyéthylèneglycol, des huiles végétales, en particulier l'huile d'olive, des esters organiques injectables, par exemple l'oléate d'éthyle. Ces compositions peuvent également contenir des adjuvants, en particulier des agents mouillants, isotonisants, émulsifiants, dispersants et stabilisants.

La stérilisation peut se faire de plusieurs façons, par exemple à l'aide d'un filtre bactériologique, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration topique peuvent être par exemple des crèmes, des pommades, des lotions ou des aérosols.

Les compositions par administration rectale sont les suppositoires ou les capsules rectales. qui contiennent outre le principe actif, des excipients tels que le beurre de cacao. des glycérides semi-synthétiques ou des polyéthyléneglycols.

Les compositions peuvent également être des aérosols. Pour l'usage sous forme d'aérosols liquides, les compositions peuvent être des solutions stériles stables ou des compositions solides dissoutes au moment de l'emploi dans de l'eau stérile apyrogène, dans du sérum ou tout autre véhicule pharmaceutiquement acceptable. Pour l'usage sous forme d'aérosols secs destinés à être directement inhalés, le principe actif est finement divisé et associé à un diluant ou véhicule solide hydrosoluble d'une granulométrie de 30 à 80 µm, par exemple le dextrane, le mannitol ou le lactose.

En thérapeutique humaine, les nouveaux dérivés de streptogramine selon l'invention sont particulièrement utiles dans le traitement des infections d'origine bactérienne. Les doses dépendent de l'effet recherché et de la durée du traitement. Le médecin déterminera la posologie qu'il estime la plus appropriée en fonction du traitement, en fonction de l'âge, du poids, du degré de l'infection et des autres facteurs propres au sujet à traiter. Généralement, les doses sont comprises entre 1 et 3 g de produit actif en 2 ou 3 prises par jour, par voie orale pour un adulte.

L'exemple suivant illustre une composition selon l'invention.

### EXEMPLE

On prépare selon la technique habituelle des comprimés dosés à 250 mg de produit actif, ayant la composition suivante:
- (16R)-16-Diméthylamino-16-désoxopristinamycine II_{A} 175 mg
- pristinamycine I_{B} 75 mg
- excipient : amidon, silice hydratée, dextrine, gélatine, stéarate de magnésium : qsp 500 mg

## Revendications

1. Un dérivé du groupe A des streptogramines **caractérisé en ce qu'**il répond à la formule générale : 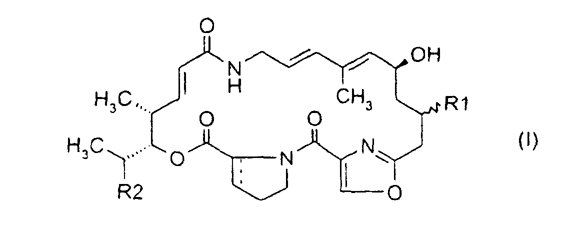 dans laquelle
R₁ est un radical -NR'R" pour lequel R' est un atome d'hydrogène ou un radical méthyle, et R" est un atome d'hydrogène, un radical alcoyle, cycloalcoyle, allyle, propynyle, benzyle, ou -OR''', R"' étant un atome d'hydrogène, un radical alcoyle, cycloalcoyle, allyle, propynyle ou benzyle, ou R" représente -NR₃R₄ , R₃ et R₄ pouvant représenter un radical méthyle, ou former ensemble avec l'atome d'azote auquel ils sont attachés un hétérocycle à 4 ou 5 chaînons saturé ou insaturé pouvant en outre contenir un autre hétéroatome choisi parmi l'azote, l'oxygène ou le soufre,
R₂ est un atome d'hydrogène ou un radical méthyle ou éthyle, et
la liaison --- représente une liaison simple ou une liaison double,
et dans laquelle sauf mention spéciale, les radicaux alcoyle sont droits ou ramifiés et contiennent 1 à 6 atomes de carbone, les radicaux cycloalcoyle contiennent 3 à 4 atomes de carbone, et la chaîne 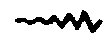 en position 16 signifie : lorsque R" est autre que -OR''' ou -NR₃R₄, l'épimère R ou les mélanges des épimères R et S dans lesquels l'épimère R est majoritaire, et lorsque R" est -OR''' ou -NR₃R₄, les épimères R et S et leurs mélanges,
ainsi que ses sels.

2. Un dérivé du groupe A des streptogramines, selon la revendication 1, **caractérisé en ce que** R₁ est un radical -NR'R" pour lequel R' est un atome d'hydrogène ou un radical méthyle, et R" est un atome d'hydrogène, un radical alcoyle, cycloalcoyle, allyle, propynyle, benzyle, ou -OR"', R''' étant un radical alcoyle contenant 1 à 6 atomes de carbone, allyle ou propynyle, ou R" représente -NR₃R₄, R₃ et R₄ pouvant représenter un radical méthyle, ou former ensemble avec l'atome d'azote auquel ils sont attachés un hétérocycle à 4 ou 5 chaînons saturé ou insaturé pouvant en outre contenir un autre hétéroatome choisi parmi l'azote, l'oxygène ou le soufre, R₂ est un atome d'hydrogène ou un radical méthyle ou éthyle, et la liaison --- représente une liaison simple ou une liaison double, ainsi que leurs sels et pour lequel la chaîne 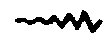 en position 16 signifie : lorsque R" est autre que -OR''' ou -NR₃R₄, l'épimère R ou les mélanges des épimères R et S dans lesquels l'épimère R est majoritaire, et lorsque R" est -OR''' ou -NR₃R₄, les épimères R et S et leurs mélanges.

3. Un dérivé du groupe A des streptogramines, selon la revendication 1 ou 2, **caractérisé en ce que** R₁ est un radical -NR'R" pour lequel R' est un atome d'hydrogène ou un radical méthyle, et R" est un atome d'hydrogène, un radical alcoyle contenant 1 à 4 atomes de carbone, cycloalcoyle, allyle, propynyle, benzyle, ou -OR''', R''' étant un radical alcoyle contenant 1 à 3 atomes de carbone, allyle ou propynyle, ou R" représente -NR₃R₄, R₃ et R₄ pouvant former ensemble avec l'atome d'azote auquel ils sont attachés un hétérocycle saturé à 5 chaînons, R₂ est un radical méthyle ou éthyle, et la liaison --- représente une liaison simple ou une liaison double, ainsi que leurs sels et pour lequel la chaîne 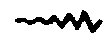 en position 16 signifie : lorsque R" est autre que -OR''' ou -NR₃R₄, l'épimère R ou les mélanges des épimères R et S dans lesquels l'épimère R est majoritaire, et lorsque R" est -OR''' ou -NR₃R₄, les épimères R et S et leurs mélanges.

4. Un dérivé du groupe A des streptogramines, selon la revendication 1, **caractérisé en ce qu'**il s'agit de la (16R)-16-diméthylamino-16-désoxopristinamycine II_{A} ainsi que ses sels

5. Un dérivé du groupe A des streptogramines, selon la revendication 1, **caractérisé en ce qu'**il s'agit de la (16R)-16-méthoxyamino-16-désoxopristinamycine II_{B} ainsi que ses sels

6. Un dérivé du groupe A des streptogramines, selon la revendication 1, **caractérisé en ce qu'**il s'agit de la(16R)-16-éthoxyamino-16-désoxopristinamycine II_{B} ainsi que ses sels

7. Un dérivé du groupe A des streptogramines, selon la revendication 1, **caractérisé en ce qu'**il s'agit de la(16R)-16-allyloxyamino-16-désoxopristinamycine II_{B} ainsi que ses sels

8. Un dérivé du groupe A des streptogramines, selon la revendication 1, **caractérisé en ce qu'**il s'agit de la(16R)-16-méthoxyamino-16-désoxopristinamycine II_{A} ainsi que ses sels.

9. Procédé de préparation d'un dérivé de streptogramine selon la revendication 1, **caractérisé en ce que** l'on fait agir une amine de formule générale :
H₂N-R'' (III)
dans laquelle R" est défini comme ci-dessus, sur une composante de la pristinamycine naturelle de formule générale : 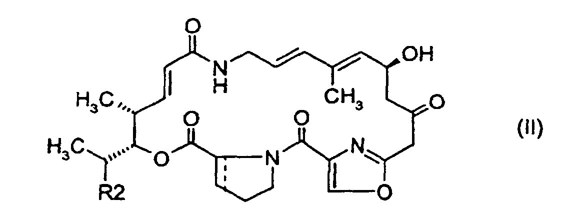 dans laquelle R₂ est défini comme dans la revendication 1, puis fait agir un agent réducteur de l'énamine (ou de l'oxime) intermédiaire obtenue et, lorsque l'on veut obtenir un dérivé de streptogramine selon la revendication 1 pour lequel R' est un radical méthyle, effectue une seconde amination réductrice, par action du formaldéhyde ou d'un dérivé générant le formaldéhyde in situ, suivie de la réduction de l'énamine intermédiaire, et transforme éventuellement le produit obtenu en un sel, et/ou sépare son épimère 16 R.

10. Procédé selon la revendication 9, **caractérisé en ce que** pour préparer un dérivé de la streptogramine selon la revendication 1 pour lequel R" est un radical -OR''', l'oxime intermédiaire de formule générale : 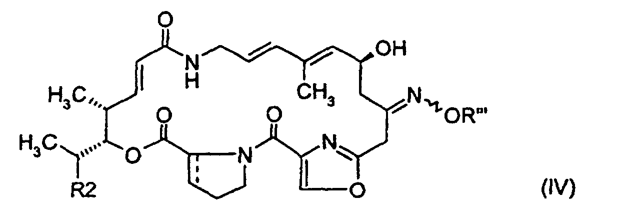 dans laquelle R₂ et R''' sont définis comme dans la revendication 1, est isolée, puis transformée par réduction en un dérivé de streptogramine selon la revendication 1 pour lequel R' est un atome d'hydrogène, pouvant être éventuellement mis en oeuvre dans l'opération subséquente d'amination-réductrice.

11. Procédé de préparation d'un dérivé de streptogramine selon la revendication 1,. **caractérisé en ce que** l'on fait agir la cétone correspondante au radical R" désiré, sur le dérivé aminé de formule générale : 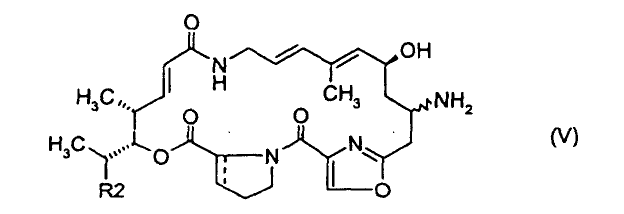 dans laquelle R₂ est défini comme précédemment, puis lorsque l'on veut obtenir un dérivé de streptogramine selon la revendication 1, pour lequel R' est un radical méthyle, effectue une seconde amination réductrice, par action du formaldéhyde ou d'un dérivé générant le formaldéhyde in situ et réduction de l'énamine intermédiaire, et transforme éventuellement le produit obtenu en un sel, et/ou sépare son épimère 16 R.

12. Associations **caractérisées en ce qu'**elles comprennent un dérivé de streptogramine du groupe A selon la revendication 1, et un dérivé de streptogramine du groupe B.

13. Associations selon la revendication 12, **caractérisées en ce que** le dérivé de streptogramine du groupe B est choisi parmi des composantes naturelles ou des composante d'hémisynthèse.

14. Associations selon la revendication 12, **caractérisées en ce que** le dérivé de streptogramine du groupe B est choisi parmi la pristinamycine IA, la pristinamycine IB, la pristinamycine IC, la pristinamycine ID, la pristinamycine IE, la pristinamycine IF, la pristinamycine IG, la virginiamycine S1, S3 ou S4, la vernamycine B ou C ou l'étamycine.

15. Associations selon la revendication 12, **caractérisées en ce que** le dérivé de streptogramine du groupe B est choisi parmi les dérivés de streptogramines de formule générale : 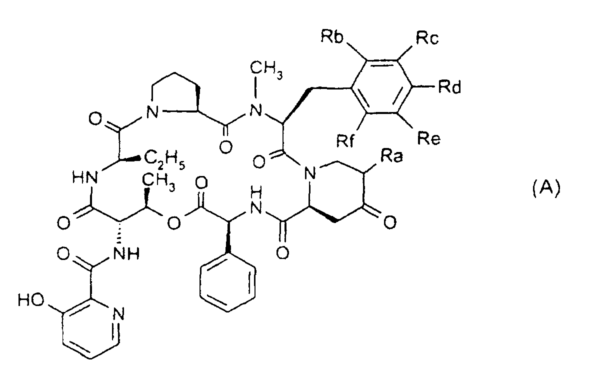 dans laquelle,
1. Rb, Rc, Re et Rf sont des atomes d'hydrogène, Rd est un atome d'hydrogène ou un radical diméthylamino, et Ra est un radical de structure -CH₂R'a pour lequel R'a est pyrrolidinyl-3thio, pipéridyl-3(ou -4)thio pouvant être substitués par alcoyle, alcoylthio substitué par 1 ou 2 hydroxysulfonyle, alcoylamino, dialcoylamino (lui même éventuellement substitué par mercapto ou dialcoylamino), ou substitué par 1 ou 2 cycles pipérazine éventuellement substitué, morpholino, thiomorpholino, pipéridino, pyrrolidinyle-1, pipéridyle-2,-3 ou -4, ou pyrrolidinyle-2 ou -3 (pouvant être substitués par alcoyle), ou bien Ra est un radical de structure =CHR'a pour lequel R'a est pyrrolidinyl-3amino, pipéridyl-3(ou -4)amino, pyrrolidinyl-3oxy, pipéridyl-3(ou -4)oxy, pyrrolidinyl-3thio, pipéridyl-3(ou -4)thio pouvant être substitués par alcoyle, ou R'a est alcoylamino, alcoyloxy ou alcoylthio substitués par 1 ou 2 hydroxysulfonyle, alcoylamino, dialcoylamino (lui même éventuellement substitué par dialcoylamino), ou par trialcoylammonio, imidazolyl-4 ou -5, ou par 1 ou 2 cycles pipérazine éventuellement substitué, morpholino, thiomorpholino, pipéridino, pyrrolidinyle-1, pipéridyle-2,-3 ou -4, ou pyrrolidinyle-2 ou -3 (pouvant être substitués par alcoyle), ou Ra est un radical quinuclidinyl-3(ou -4)thiométhyle ou bien
2. Ra est un atome d'hydrogène et
a) soit Rb, Re et Rf sont des atomes d'hydrogène, Rd est un radical -NHCH₃ ou -N(CH₃)₂ et Rc est un atome de chlore ou de brome, ou représente un radical alcényle contenant 3 à 5 atomes de carbone [si Rd est -N(CH₃)₂],
b) soit Rb, Rd, Re et Rf représentent un atome d'hydrogène et Rc est un halogène, ou un radical aminomonoalkyle, aminodialkyle, alcoyloxy, trifluorométhyloxy, thioalcoyle,alcoyle en C₁ à C₃ ou trihalogénométhyle
c) soit Rb, Rc, Re et Rf représentent un atome d'hydrogène et Rd est un halogène, ou un radical éthylamino, diéthylamino ou méthyléthylamino, alcoyloxy ou trifluorométhyloxy, thioalkyle, alcoyle en C₁ à C₆, aryle ou trihalogénométhyle
d) soit Rb, Re et Rf représentent un atome d'hydrogène et Rc est halogène ou un radical aminomonoalkyle ou aminodialkyle, alcoyloxy ou trifluorométhyloxy, thioalkyle, alcoyle en C₁ à C₃, et Rd est halogène ou un radical amino, aminomonoalkyle ou aminodialkyle, alcoyloxy ou trifluorométhyloxy, thioalkyle, alcoyle en C₁ à C₆ ou trihalogénométhyle,
e) soit Rc, Re et Rf représentent un atome d'hydrogène et Rb et Rd représentent un radical méthyle.

16. Composition pharmaceutique **caractérisée en ce qu'**elle contient au moins un dérivé de la streptogramine selon l'une des revendications 1 à 8, éventuellement en association avec un dérivé du groupe B des streptogramines et/ou éventuellement en association avec tout diluant ou adjuvant compatible et pharmaceutiquement acceptable.

## Patentansprüche

1. Ein Derivat der Gruppe A der Streptogramine, **dadurch gekennzeichnet, dass** es der allgemeinen Formel: 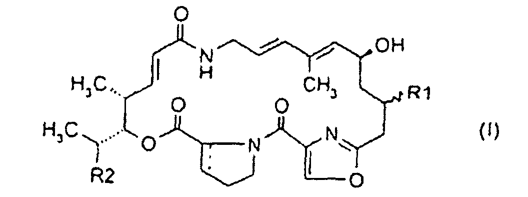 entspricht, worin
R₁ ein Rest -NR'R" ist, für den R' ein Wasserstoffatom oder ein Methylrest ist und R" ein Wasserstoffatom, ein Alkyl-, Cycloalkyl-, Allyl-, Propinyl-, Benzyl- oder -OR"'-Rest ist, wobei R''' ein Wasserstoffatom, ein Alkyl-, Cycloalkyl-, Allyl-, Propinyl- oder Benzylrest ist, oder R" -NR₃R₄ darstellt, wobei R₃ und R₄ einen Methylrest darstellen können oder zusammen mit dem Stickstoffatom, an das sie gebunden sind, einen gesättigten oder ungesättigten Heterocyclus mit 4 oder 5 Ringgliedern, der außerdem ein weiteres Heteroatom, das unter Stickstoff, Sauerstoff oder Schwefel ausgewählt ist, enthalten kann, bilden können,
R₂ ein Wasserstoffatom oder ein Methyl- oder Ethylrest ist und
die Bindung --- eine Einfachbindung oder eine Doppelbindung darstellt,
und worin außer bei spezieller Angabe die Alkylreste gerade oder verzweigt sind und 1 bis 6 Kohlenstoffatome enthalten, die Cycloalkylreste 3 bis 4 Kohlenstoffatome enthalten und die Kette in 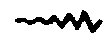 Position 16 bedeutet: das R-Epimer oder die Gemische von R- und S-Epimer, in denen das R-Epimer überwiegt, wenn R" von -OR''' oder -NR₃R₄ verschieden ist, und das R- und S-Epimer und ihre Gemische, wenn R" -OR''' oder -NR₃R₄ ist,
sowie seine Salze.

2. Ein Derivat der Gruppe A der Streptogramine gemäß Anspruch 1, **dadurch gekennzeichnet, dass** R₁ ein Rest -NR'R" ist, für den R' ein Wasserstoffatom oder ein Methylrest ist und R" ein Wasserstoffatom, ein Alkyl-, Cycloalkyl-, Allyl-, Propinyl-, Benzyl- oder -OR'''-Rest ist, wobei R"' ein Rest Alkyl mit 1 bis 6 Kohlenstoffatomen, Allyl oder Propinyl ist, oder R" -NR₃R₄ darstellt, wobei R₃ und R₄ einen Methylrest darstellen können oder zusammen mit dem Stickstoffatom, an das sie gebunden sind, einen gesättigten oder ungesättigten Heterocyclus mit 4 oder 5 Ringgliedern, der außerdem ein weiteres Heteroatom, das unter Stickstoff, Sauerstoff oder Schwefel ausgewählt ist, enthalten kann, bilden können, R₂ ein Wasserstoffatom oder ein Methyl- oder Ethylrest ist und die Bindung --- eine Einfachbindung oder eine Doppelbindung darstellt, sowie seine Salze und für das die Kette in 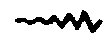 Position 16 bedeutet: das R-Epimer oder die Gemische von R- und S-Epimer, in denen das R-Epimer überwiegt, wenn R" von -OR''' oder -NR₃R₄ verschieden ist, und das R- und S-Epimer und ihre Gemische, wenn R" -OR''' oder -NR₃R₄ ist.

3. Ein Derivat der Gruppe A der Streptogramine gemäß Anspruch 1 oder 2, **dadurch gekennzeichnet, dass** R₁ ein -NR'R"-Rest ist, für den R' ein Wasserstoffatom oder ein Methylrest ist und R" ein Wasserstoffatom, ein Rest Alkyl mit 1 bis 4 Kohlenstoffatomen, Cycloalkyl, Allyl, Propinyl, Benzyl oder -OR''' ist, wobei R"' ein Rest Alkyl mit 1 bis 3 Kohlenstoffatomen, Allyl oder Propinyl ist, oder R" -NR₃R₄ darstellt, wobei R₃ und R₄ zusammen mit dem Stickstoffatom, an das sie gebunden sind, einen gesättigten Heterocyclus mit 5 Ringgliedern bilden können, R₂ ein Methyl- oder Ethylrest ist und die Bindung --- eine Einfachbindung oder eine Doppelbindung darstellt, sowie seine Salze und für das die 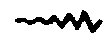 Kette in Position 16 bedeutet: das R-Epimer oder die Gemische von R- und S-Epimer, in denen das R-Epimer überwiegt, wenn R" von -OR''' oder -NR₃R₄ verschieden ist, und das R- und S-Epimer und ihre Gemische, wenn R" -OR''' oder -NR₃R₄ ist.

4. Ein Derivat der Gruppe A der Streptogramine gemäß Anspruch 1, **dadurch gekennzeichnet, dass** es sich um (16R)-16-Dimethylamino-16-desoxopristinamycin II_{A} sowie seine Salze handelt.

5. Ein Derivat der Gruppe A der Streptogramine gemäß Anspruch 1, **dadurch gekennzeichnet, dass** es sich um (16R)-16-Methoxyamino-16-desoxopristinamycin II_{B} sowie seine Salze handelt.

6. Ein Derivat der Gruppe A der Streptogramine gemäß Anspruch 1, **dadurch gekennzeichnet, dass** es sich um (16R)-16-Ethoxyamino-16-desoxopristinamycin II_{B} sowie seine Salze handelt.

7. Ein Derivat der Gruppe A der Streptogramine gemäß Anspruch 1, **dadurch gekennzeichnet, dass** es sich um (16R)-16-Allyloxyamino-16-desoxopristinamycin II_{B} sowie seine Salze handelt.

8. Ein Derivat der Gruppe A der Streptogramine gemäß Anspruch 1, **dadurch gekennzeichnet, dass** es sich um (16R)-16-Methoxyamino-16-desoxopristinamycin II_{A} sowie seine Salze handelt.

9. Verfahren zur Herstellung eines Streptograminderivats gemäß Anspruch 1, **dadurch gekennzeichnet, dass** man ein Amin der allgemeinen Formel:
H₂N-R" (III)
worin R" wie oben definiert ist, auf eine Komponente des natürlichen Pristinamycins der allgemeinen Formel: 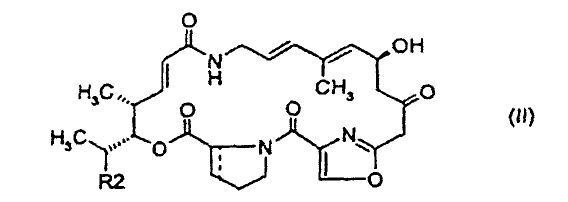 worin R₂ wie in Anspruch 1 definiert ist, einwirken lässt, dann ein Mittel, das das erhaltene Enamin- (oder Oxim-)zwischenprodukt reduziert, einwirken lässt und, wenn man ein Streptograminderivat gemäß Anspruch 1, für das R' ein Methylrest ist, erhalten will, eine zweite reduzierende Aminierung durch Einwirken von Formaldehyd oder eines Derivats, das in situ Formaldehyd erzeugt, und anschließende Reduktion des Enaminzwischenprodukts ausführt und gegebenenfalls das erhaltene Produkt in ein Salz umwandelt und/oder sein 16R-Epimer abtrennt.

10. Verfahren gemäß Anspruch 9, **dadurch gekennzeichnet, dass** zur Herstellung eines Streptograminderivats gemäß Anspruch 1, für das R" ein -OR'''-Rest ist, das Oximzwischenprodukt der allgemeinen Formel: 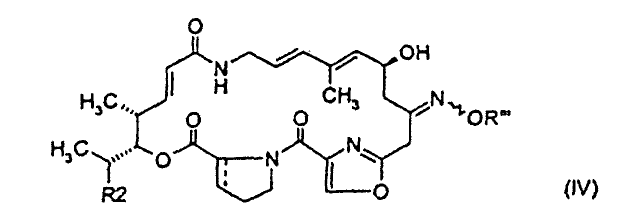 worin R₂ und R''' wie in Anspruch 1 definiert sind, isoliert wird, dann durch Reduktion in ein Streptograminderivat gemäß Anspruch 1, für das R' ein Wasserstoffatom ist, umgewandelt wird, das gegebenenfalls beim nachfolgenden Arbeitsschritt der reduzierenden Aminierung verwendet werden kann.

11. Verfahren zur Herstellung eines Streptograminderivats gemäß Anspruch 1, **dadurch gekennzeichnet, dass** man das dem gewünschten Rest R" entsprechende Keton auf das Aminderivat der allgemeinen Formel: 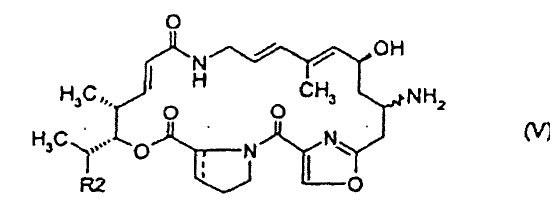 worin R₂ wie zuvor definiert ist, einwirken lässt, dann, wenn man ein Streptograminderivat gemäß Anspruch 1, für das R' ein Methylrest ist, erhalten will, eine zweite reduzierende Aminierung durch Einwirken von Formaldehyd oder eines Derivats, das in situ Formaldehyd erzeugt, und eine Reduktion des Enaminzwischenprodukts ausführt und gegebenenfalls das erhaltene Produkt in ein Salz umwandelt und/oder sein 16R-Epimer abtrennt.

12. Kombinationen, **dadurch gekennzeichnet, dass** sie ein Derivat eines Streptogramins der Gruppe A gemäß Anspruch 1 und ein Derivat eines Streptogramins der Gruppe B umfassen.

13. Kombinationen gemäß Anspruch 12, **dadurch gekennzeichnet, dass** das Derivat eines Streptogramins der Gruppe B unter natürlichen Komponenten oder halbsynthetischen Komponenten ausgewählt ist.

14. Kombinationen gemäß Anspruch 12, **dadurch gekennzeichnet, dass** das Derivat eines Streptogramins der Gruppe B ausgewählt ist unter Pristinamycin IA, Pristinamycin IB, Pristinamycin IC, Pristinamycin ID, Pristinamycin IE, Pristinamycin IF, Pristinamycin IG, Virginiamycin S1, S3 oder S4, Vernamycin B oder C oder Etamycin.

15. Kombinationen gemäß Anspruch 12, **dadurch gekennzeichnet, dass** das Derivat eines Streptogramins der Gruppe B ausgewählt ist unter den Streptograminderivaten der allgemeinen Formel: 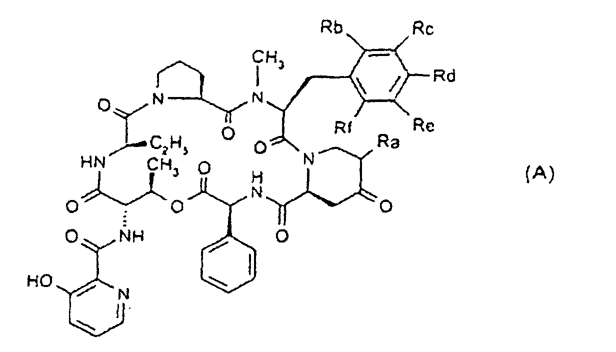 worin
1. Rb, Rc, Re und Rf Wasserstoffatome sind, Rd ein Wasserstoffatom oder ein Dimethylaminorest ist und Ra ein Rest mit einer Struktur -CH₂R'a ist, für den R'a 3-Pyrrolidinylthio, 3-(oder 4-)Piperidylthio, die substituiert sein können mit Alkyl, Alkylthio, substituiert mit 1 oder 2 Hydroxysulfonyl, Alkylamino, Dialkylamino (selbst gegebenenfalls substituiert mit Mercapto oder Dialkylamino) oder substituiert mit 1 oder 2 Ringen Piperazin, gegebenenfalls substituiert, Morpholino, Thiomorpholino, Piperidino, 1-Pyrrolidinyl, 2-, 3- oder 4-Piperidyl oder 2- oder 3-Pyrrolidinyl (die mit Alkyl substituiert sein können) ist, oder aber Ra ein Rest mit einer Struktur =CHR'a ist, für den R'a 3-Pyrrolidinylamino, 3-(oder 4-)Piperidylamino, 3-Pyrrolidinyloxy, 3-(oder 4-)Piperidyloxy, 3-Pyrrolidinylthio, 3-(oder 4-)Piperidylthio, die mit Alkyl substituiert sein können, ist oder R'a Alkylamino, Alkyloxy oder Alkylthio, substituiert mit 1 oder 2 Hydroxysulfonyl, Alkylamino, Dialkylamino (selbst gegebenenfalls substituiert mit Dialkylamino) oder mit Trialkylammonio, 4- oder 5-Imidazolyl oder mit 1 oder 2 Ringen Piperazin, gegebenenfalls substituiert, Morpholino, Thiomorpholino, Piperidino, 1-Pyrrolidinyl, 2-, 3- oder 4-Piperidyl oder 2- oder 3-Pyrrolidinyl (die mit Alkyl substituiert sein können), ist oder Ra ein 3-(oder 4-)Chinolidinylthiomethylrest ist oder aber
2. Ra ein Wasserstoffatom ist und
a) entweder Rb, Re und Rf Wasserstoffatome sind, Rd ein -NHCH₃- oder -N(CH₃)₂-Rest ist und Rc ein Chlor- oder Bromatom ist oder einen Alkenylrest mit 3 bis 5 Kohlenstoffatomen darstellt [wenn Rd -N(CH₃)₂ ist],
b) oder Rb, Rd, Re und Rf ein Wasserstoffatom darstellen und Rc ein Halogen oder ein Aminomonoalkyl-, Aminodialkyl-, Alkyloxy-, Trifluormethyloxy-, Thioalkyl-, C₁- bis C₃-Alkyl- oder Trihalogenmethylrest ist,
c) oder Rb, Rc, Re und Rf ein Wasserstoffatom darstellen und Rd ein Halogen oder ein Ethylamino-, Diethylamino- oder Methylethylamino-, Alkyloxy- oder Trifluormethyloxy-, Thioalkyl-, C₁- bis C₆-Alkyl-, Aryl- oder Trihalogenmethylrest ist,
d) oder Rb, Re und Rf ein Wasserstoffatom darstellen und Rc Halogen oder ein Aminomonoalkyl- oder Aminodialkyl-, Alkyloxy- oder Trifluormethyloxy-, Thioalkyl-, C₁- bis C₃-Alkylrest ist und Rd Halogen oder ein Amino-, Aminomonoalkyl- oder Aminodialkyl-, Alkyloxy- oder Trifluormethyloxy-, Thioalkyl-, C₁- bis C₆-Alkyl- oder Trihalogenmethylrest sind,
e) oder Rc, Re und Rf ein Wasserstoffatom darstellen und Rb und Rd einen Methylrest darstellen.

16. Pharmazeutische Zusammensetzung, **dadurch gekennzeichnet, dass** sie wenigstens ein Streptograminderivat gemäß einem der Ansprüche 1 bis 8 enthält, gegebenenfalls in Kombination mit einem Derivat der Gruppe B der Streptogramine und/oder gegebenenfalls in Kombination mit jedem Verdünnungsmittel oder Zusatzmittel, das kompatibel und pharmazeutisch unbedenklich ist.

## Claims

1. Derivative of group A streptogramins, **characterized in that** it corresponds to the general formula: 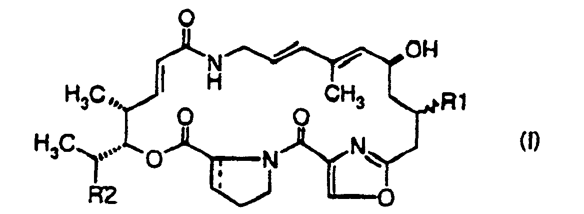 in which
R₁ is a radical -NR'R'' for which R' is a hydrogen atom or a methyl radical, and R'' is a hydrogen atom or an alkyl, cycloalkyl, allyl, propynyl, benzyl or -OR''' radical, R''' being a hydrogen atom or an alkyl, cycloalkyl, allyl, propynyl or benzyl radical, or R'' represents -NR₃R₄, it being possible for R₃ and R₄ to represent a methyl radical, or to form together with the nitrogen atom to which they are attached a saturated or unsaturated 4- or 5-membered heterocycle which may, in addition, contain another heteroatom chosen from nitrogen, oxygen or sulphur,
R₂ is a hydrogen atom or a methyl or ethyl radical, and
the bond --- represents a single bond or a double bond,
and in which unless otherwise stated, the alkyl radicals are straight or branched and contain 1 to 6 carbon atoms; the cycloalkyl radicals contain 3 to 4 carbon atoms; the chain 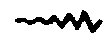 at the 16-position means: when R'' is other than -OR''' or -NR₃R₄, the R epimer or mixtures of the R and S epimers in which the R epimer is predominant, and when R'' is -OR''' or -NR₃R₄, the R and S epimers and mixtures thereof,
as well as its salts.

2. Derivative of group A streptogramins according to Claim 1, **characterized in that** R₁ is a radical -NR'R'' for which R' is a hydrogen atom or a methyl radical, and R'' is a hydrogen atom, an alkyl, cycloalkyl, allyl, propynyl, benzyl or -OR''' radical, R''' being an alkyl radical containing 1 to 6 carbon atoms, an allyl or propynyl radical, or R'' represents -NR₃R₄, it being possible for R₃ and R₄ to represent a methyl radical, or to form together with the nitrogen atom to which they are attached a saturated or unsaturated 4- or 5-membered heterocycle which may, in addition, contain another heteroatom chosen from nitrogen, oxygen or sulphur, R₂ is a hydrogen atom or a methyl or ethyl radical, and the bond --- represents a single bond or a double bond, as well as their salts and in which the chain 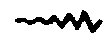 at the 16-position means: when R" is other than -OR''' or -NR₃R₄, the R epimer or mixtures of the R and S epimers in which the R epimer is predominant, and when R'' is -OR''' or -NR₃R₄, the R and S epimers and mixtures thereof.

3. Derivative of group A streptogramins according to Claim 1 or 2, **characterized in that** R₁ is a radical -NR'R'' for which R' is a hydrogen atom or a methyl radical, and R'' is a hydrogen atom, an alkyl radical containing 1 to 4 carbon atoms, a cycloalkyl, allyl, propynyl, benzyl or -OR''' radical, R''' being an alkyl radical containing 1 to 3 carbon atoms, or an allyl or propynyl radical, or R'' represents -NR₃R₄, it being possible for R₃ and R₄ to form together with the nitrogen atom to which they are attached a 5-membered saturated heterocycle, R₂ is a methyl or ethyl radical, and the bond --- represents a single bond or a double bond, as well as their salts and in which the chain 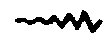 at the 16-position means: when R'' is other than -OR''' or -NR₃R₄, the R epimer or mixtures of the R and S epimers in which the R epimer is predominant, and when R'' is -OR''' or -NR₃R₄, the R and S epimers and mixtures thereof.

4. Derivative of group A streptogramins according to Claim 1, **characterized in that** it is (16R)-16-dimethylamino-16-deoxopristinamycin II_{A} as well as its salts.

5. Derivative of group A streptogramins according to Claim 1, **characterized in that** it is (16R)-16-methoxyamino-16-deoxopristinamycin II_{B} as well as its salts.

6. Derivative of group A streptogramins according to Claim 1, **characterized in that** it is (16R)-16-ethoxyamino-16-deoxopristinamycin II_{B} as well as its salts.

7. Derivative of group A streptogramins according to Claim 1, **characterized in that** it is (16R)-16-allyloxyamino-16-deoxopristinamycin II_{B} as well as its salts.

8. Derivative of group A streptogramins according to Claim 1, **characterized in that** it is (16R)-16-methoxyamino-16-deoxopristinamycin II_{A} as well as its salts.

9. Process for preparing a streptogramin derivative according to Claim 1, **characterized in that** an amine of general formula:
H₂N-R'' (III)
in which R'' is as defined above, is reacted with a component of natural pristinamycin of general formula: 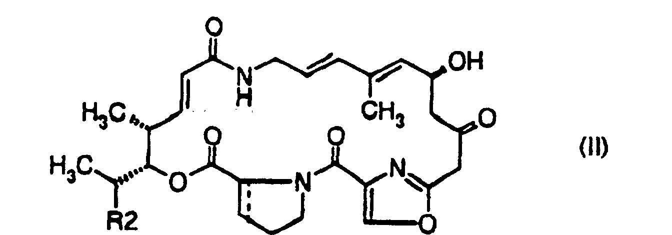 in which R₂ is as defined in Claim 1, and then an agent for reducing the intermediate enamine (or oxime) obtained is caused to react and, when it is desired to obtain a streptogramin derivative according to Claim 1 for which R' is a methyl radical, a second reductive amination is carried out by the action of formaldehyde or of a derivative generating formaldehyde in situ, followed by the reduction of the intermediate enamine, the product obtained is optionally converted to a salt, and/or its 16R epimer is separated.

10. Process according to Claim 9, **characterized in that** to prepare a streptogramin derivative according to Claim 1 for which R'' is a radical -OR''', the intermediate oxime of general formula: 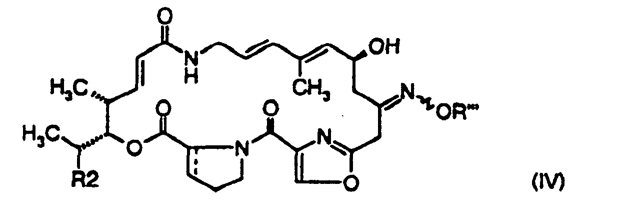 in which R₂ and R''' are as defined in Claim 1, is isolated, and then converted by reduction to a streptogramin derivative according to claim 1 for which R' is a hydrogen atom, which may be optionally used in the subsequent reductive amination operation.

11. Process for preparing a streptogramin derivative according to Claim 1, **characterized in that** the ketone corresponding to the desired R'' radical is reacted with the amine-containing derivative of general formula: 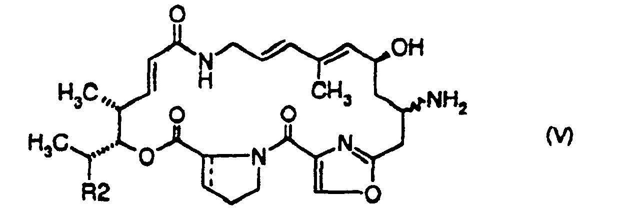 in which R₂ is as defined above, and then when it is desired to obtain a streptogramin derivative according to claim 1, for which R' is a methyl radical, a second reductive amination is carried out, by the action of formaldehyde or of a derivative generating formaldehyde in situ and the intermediate enamine is reduced, and the product obtained is optionally converted to a salt, and/or its 16R epimer is separated.

12. Combinations **characterized in that** they comprise a group A streptogramin derivative according to Claim 1 and a group B streptogramin derivative.

13. Combinations according to Claim 12, **characterized in that** the group B streptogramin derivative is chosen from natural components or semisynthetic components.

14. Combinations according to Claim 12, **characterized in that** the group B streptogramin derivative is chosen from pristinamycin I_{A}, pristinamycin I_{B}, pristinamycin I_{C}, pristinamycin I_{D}, pristinamycin I_{E}, pristinamycin I_{F}, pristinamycin I_{G}, virginiamycin S₁, S₃ or S₄, vernamycin B or C or etamycin.

15. Combinations according to Claim 12, **characterized in that** the group B streptogramin derivative is chosen from the streptogramin derivatives of general formula: 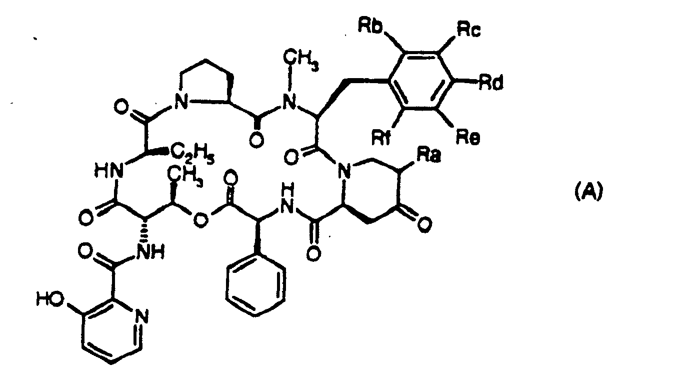 in which,
1. Rb, Rc, Re and Rf are hydrogen atoms, Rd is a hydrogen atom or a dimethylamino radical, and Ra is a radical of structure -CH₂R'a for which R'a is 3-pyrrolidinylthio or 3- or 4-piperidylthio which may be substituted with alkyl, or alkylthio substituted with 1 or 2 hydroxysulphonyl, alkylamino, dialkylamino (itself optionally substituted with mercapto or dialkylamino), or substituted with 1 or 2 optionally substituted piperazine rings, morpholino, thiomorpholino, piperidino, 1-pyrrolidinyl, 2-, 3- or 4-piperidyl or 2- or 3-pyrrolidinyl (which may be substituted with alkyl), or alternatively Ra is a radical of structure =CHR'a for which R'a is 3-pyrrolidinylamino, 3- or 4-piperidylamino, 3-pyrrolidinyloxy, 3- or 4-piperidyloxy, 3-pyrrolidinylthio, 3- or 4-piperidylthio which may be substituted with alkyl, or R'a is alkylamino, alkyloxy or alkylthio substituted with 1 or 2 hydroxysulphonyl, alkylamino, dialkylamino (itself optionally substituted with dialkylamino), or with trialkylammonio, 4- or 5-imidazolyl, or with 1 or 2 optionally substituted piperazine rings, morpholino, thiomorpholino, piperidino, 1-pyrrolidinyl, 2-, 3- or 4-piperidyl or 2- or 3-pyrrolidinyl (which may be substituted with alkyl), or
Ra is a 3- or 4-quinuclidinylthiomethyl radical, or alternatively
2. Ra is a hydrogen atom and
a) either Rb, Re and Rf are hydrogen atoms, Rd is a radical -NHCH₃ or -N(CH₃)₂ and Rc is a chlorine or bromine atom, or represents an alkenyl radical containing 3 to 5 carbon atoms [if Rd is -N(CH₃)₂],
b) or Rb, Rd, Re and Rf represent a hydrogen atom and Rc is a halogen, or an aminomonoalkyl, aminodialkyl, alkyloxy, trifluoromethyloxy, thioalkyl, C₁ to C₃ alkyl or trihalomethyl radical,
c) or Rb, Rc, Re and Rf represent a hydrogen atom and Rd is a halogen, or an ethylamino, diethylamino or methylethylamino, alkyloxy or trifluoromethyloxy, thioalkyl, C₁ to C₆ alkyl, aryl or trihalomethyl radical,
d) or Rb, Re and Rf represent a hydrogen atom and Rc is halogen or an aminomonoalkyl or aminodialkyl, alkyloxy or trifluoromethyloxy, thioalkyl or C₁ to C₃ alkyl radical, and Rd is halogen or an amino, aminomonoalkyl or aminodialkyl, alkyloxy or trifluoromethyloxy, thioalkyl, C₁ to C₆ alkyl or trihalomethyl radical,
e) or Rc, Re and Rf represent a hydrogen atom and Rb and Rd represent a methyl radical.

16. Pharmaceutical composition, **characterized in that** it contains at least one streptogramin derivative according to one of Claims 1 to 8, optionally in combination with a group B streptogramin derivative, and/or optionally in combination with any compatible and pharmaceutically acceptable diluent or adjuvant.
